# EUROPÄISCHE PATENTSCHRIFT

(11) **EP 0 835 093 B1**
(45) Veröffentlichungstag und Bekanntmachung des Hinweises auf die Patenterteilung: **10.03.2004**
(21) Anmeldenummer: 96920808.1
(22) Anmeldetag: 07.06.1996
(51) Int. Cl.: A61K 7/13

(54) **HAARFÄRBEMITTEL MIT MINDESTENS EINEM PFLEGESTOFF**
HAIR DYE AGENTS WITH AT LEAST ONE CONDITIONER
AGENTS DE COLORATION CAPILLAIRES COMPORTANT AU MOINS UN PRINCIPE ACTIF TRAITANT

(30) Priorität: 26.06.1995 DE 19522569; 02.05.1996 DE 19617490
(43) Veröffentlichungstag der Anmeldung: 15.04.1998
(73) Patentinhaber: Hans Schwarzkopf & Henkel GmbH & Co. KG, 22763 Hamburg (DE)
(72) Erfinder: DEUTZ, Herbert, D-53925 Kall (DE); AKRAM, Mustafa, D-22457 Hamburg (DE); KLEEN, Astrid, D-22081 Hamburg (DE)
(74) Vertreter: Foitzik, Joachim Kurt
(86) Internationale Anmeldenummer: PCT/EP1996/002482
(87) Internationale Veröffentlichungsnummer: WO 1997/001323

(56) Entgegenhaltungen:
- EP-A- 0 026 473
- WO-A-93/19725
- DE-A- 3 929 333
- DE-A- 4 136 997
- US-A- 4 268 264

## Beschreibung

Die Erfindung betrifft Haarbehandlungsmittel in Form von Färbemitteln für Humanhaare auf Basis von direktziehenden Farbstoffen und/oder Oxidationsfarbstoffvorprodukten mit in der Kosmetik üblichen Bestandteilen mit mindestens einem Pflegestoff.

Zu den erfindungsgemäßen Haarbehandlungsmitteln gehören alle Mittel, die es gestatten, die ursprüngliche Farbe des Haares zu verändern, wie diese z.B. in dem Buch Kosmetik von Wilfried Umbach, Georg Thieme Verlag Stuttgart, New York, 1988 auf den Seiten 284 bis 291 sowie in der dort zitierten Literatur auf der Seite 297 mit der Überschrift "4.5 Mittel zur Farbänderung", sowie Cosmetics Science and Technology, Editor Edward Sagarin, Interscience Publishery, Inc., New York und London, 1957, Seiten 479 bis 530; Karlheinz Schrader, Grundlagen und Rezepturen der Kosmetika, 2. verbesserte und erweiterte Auflage, Hüthig Buch Verlag, Heidelberg 1989, Seite 782 bis 815; Hair Coloring, Rev. Prog. Coloration, Vol. 15, 52ff. (1985); J.F. Corbett, The Chemistry of Synthetic Dyes, Vol. 5, Edited by Venkataraman, Academic Press, New York and London, 1971 beschrieben sind.

Direktziehende Farbstoffe werden üblicherweise allein oder im Gemisch für Haarbehandlungsmittel in Form von Haartönungsmitteln verarbeitet, wie dies auch in vorstehender Literatur näher ausgeführt wird.

Eine besondere Bedeutung für die Färbung von Haaren besitzen die sogenannten Oxidationshaarfärbemittel, die durch oxidative Kupplung von Entwicklerkomponenten (wie z.B. p-Phenylendiaminen, p-Aminophenolen oder p-Diaminopyridinen) mit Kupplungskomponenten (wie z.B. Phenolen, Resorcinen, m-Aminophenolen, m-Phenylendiaminen, Naphtholen oder Pyrazolonen) entstehen. Unter den anwendungstechnischen Randbedingungen (tiefe Färbetemperatur und kurze Färbedauer) ergeben sie intensive Färbungen mit sehr guten Echtheiten. In der Pelzfärbung spielen die Oxidationsfärbemittel ebenfalls eine bedeutende Rolle. Gute Oxidationshaarfärbemittel müssen in erster Linie die folgenden anwendungstechnischen Voraussetzungen erfüllen: Sie müssen bei der oxidativen Kupplung mit dem jeweiligen Kupplungs- bzw. Entwicklungskomponenten die gewünschte Färbung, die ein gutes Aufziehund Ausgleichsvermögen auf Haar bzw. Pelz besitzen soll, in ausreichender Intensität ergeben. Die gebildeten Farbstoffe müssen allgemein stabil und speziell waschecht, lichtecht, schweissecht und thermostabil sein. Insbesondere dürfen sie unter den Tragebedingungen nicht zu Farbverschiebungen der ursprünglichen Nuance neigen. Darüber hinaus sollen sie toxikologisch und dermatologisch unbedenklich sein.

Voraussetzung für eine oxidative Färbung von Haaren jedoch ist, daß die Oxidationsfarbstoffvorprodukte in das Haar eindringen können. Um dieses zu gewährleisten, werden zu den Haarfärbemitteln Alkalien, vorzugsweise Ammoniak, zugesetzt. Durch das Oxidationsmittel, das nicht nur zur Kupplung der Farbstoffvorprodukte dient, sondern auch das Melanin des Haares zerstört, und durch das Alkali, insbesondere durch das Ammoniak, wird das Haar bei einer Färbung immer geschädigt. Daher versucht man, nach der Oxidationsfärbung der Haare, in einem weiteren Behandlungsschritt die Schädigung der Haare zu mindern. Dies geschieht z.B. durch Behandlung der Haare mit einem Pflegestoffe enthaltenden Shampoo, Spülungen oder Kuren.

Die EP-A-0 026 473 offenbart ein Oxidationshaarfärbemittel, welches zur Erhöhung der Farbtiefe Aminosäuren, wie beispielsweise serin enthalten kann.

Die JP-A-348612 offenbart enzymatische Haarfärbemittel.

In der DE 4408506 ist ein Haarfärbemittel beschrieben, welches Phospholipid EFA [Tri-(3-N,N-Dimethyl-N-linolenamidopropyl-2-hydroxy-ammoniumpropyl)-Phosphorsäureestertrichlorid] als Pflegemittel enthält. Dieser Pflegestoff bewirkt eine verbesserte Naßkämmbarkeit der gefärbten Keratinfasern gegenüber mit bekannten Färbemitteln behandelten Haaren. Es wird während des Färbevorgangs eine positive Wirkung, jedoch lediglich auf den äußeren Cutikula-Bereich, hervorgerufen. Jedoch wird keine innere Strukturverbesserung der Haare bewirkt.

Daher besteht die dringende Aufgabe, Haarbehandlungsmittel in Form von Färbemitteln für Humanhaare auf Basis von direktziehenden und/oder Oxidationsfarbstoffvorprodukten mit Pflegestoffen zu entwickeln, die den Zustand des Haares schon während der Färbung weniger strapazieren bzw. sogar die Haarstruktur von innen her verbessern.

Die Aufgabe der Erfindung wird durch die in den Patentansprüchen 1 bis 28 genannten Haarfärbemittel gelöst. Die erfindungsgemäß enthaltenen Pflegestoffe der allgemeinen Formel (I) sollen in die zu färbenden Haare eindringen und im pH-Bereich von 2 bis 12 stabil sein. Hierdurch soll neben verbesserter Kämmbarkeit und verbessertem Griff vor allem eine deutlich meßbare und bleibende Strukturstabilisierung der Haare, insbesondere an geschädigten Stellen, bewirkt werden.

Der besondere Vorteil der vorliegenden Erfindung besteht darin, daß die mit dem hier beschriebenen Haarfärbemittel behandelten Haare die vorstehend erläuterte, übliche Nachbehandlung der gefärbten Haare nicht benötigen.

Überraschend ist, daß durch die Haarfärbemittel der Erfindung schon während des Färbevorganges eine nachweisbare Strukturverbesserung der Haare eintritt.

Haarbehandlungsmittel der Erfindung können in Form von wäßrigen, pulverförmigen, emulgierten, dispergierten Haarfärbemittel vorliegen. Beispiele hierfür sind Lösungen, Cremes, Emulsionen, Gele, Schaumaerosole oder breiartige Zubereitungen.

Die Haarfärbemittel der Erfindung können neben mindestens einer Verbindung der allgemeinen Formel (I) in einer Menge von 0.01 bis 10 Gew.-% zusätzlich Tryptophan oder eine andere Aminosäure aus der Reihe Glycin, Alanin, Valin, Leucin, Isoleucin, Norleucin, Phenylalanin, Arginin, Histidin, Prolin, Hydroxyprolin, Cystin, Cystein, Methionin, Asparaginsäure, Glutaminsäure und Cysteinsäure und/oder deren Gemische in Mengen von 0.01 bis 5 Gew.-% enthalten.

In einer speziellen Ausführungsform kann zusätzlich Tryptophan bis zu der gleichen Gewichtsmenge der enthaltenen Verbindungen der allgemeinen Formel (I) anwesend sein.

Bevorzugte Pflegestoffe der Erfindung gemäß der allgemeinen Formel (I) sind in der Tabelle I angegeben.

**Tabelle I:**

| | **R**^{**1**} | **R**^{**2**} | **R**^{**3**} | **R**^{**4**} | **R**^{**5**} | **R**^{**6**} |
|---|---|---|---|---|---|---|
| **Serin** | OH | H | H | OH | | |
| **Threonin** | OH | H | CH₃ | OH | | |
| **Tyrosin** | OH | H | H | p-Hydroxyphenyl | | |
| **Lysin** | OH | H | H | -(CH₂)₃-NH₂ | | |
| **Serin-Alkylester** | C ₁₋₃ Alkoxy | H | H | OH | | |
| **Threonin-Alkylester** | C ₁₋₃ Alkoxy | H | CH₃ | OH | | |
| **Phosphoserin** | OH | H | H | H₂PO₃ | | |
| **Phosphothreonin** | OH | H | CH₃ | H₂PO₃ | | |
| **Serin-Dimer** | Formel (II) | H | H | OH | OH | H |

Darüber hinaus können die Haarfärbemittel der Erfindung neben mindestens einem Pflegestoff der allgemeinen Formel (I) zusätzlich einen oder mehrere Pflegestoffe, wie z.B. Panthenol, Tocopherol, Weizenproteine, Lecithine, Keratin- und Seidenhydrolysate, Phospholipide, Ceramide, Pseudoceramide, Phytosterole, enthalten. Diese zusätzlichen Pflegestoffe können im Haarfärbemittel in Mengen von 0.01 bis 10 Gew.-% vorliegen.

Proteine und insbesondere Weizenproteine sind u.a. im Römpp Chemie Lexikon, 9. Auflage, Bd. 5 auf den Seiten 3650 bis 3657 und Bd. 6 auf den Seiten 5029 bis 5030 beschrieben. Die Verwendung und Wirkung von Tocopherolen ist u.a. im Römpp Chemie Lexikon, 9. Auflage, Bd. 6 auf den Seiten 4637 bis 4639 beschrieben. Aufbau und Eigenschaften von Phytosterolen sind z.B. in Parfümerie & Kosmetik, 75. Jahrgang (1994) auf den Seiten 755 bis 758 beschrieben. Lecithine und ihre Eigenschaften sind z.B. von S.A. Riethmayer in SÖFW, 121. Jahrgang (1995) auf den Seiten 367 bis 72 beschrieben. Ceramide und Pseudoceramide sind u.a. im Römpp Chemie Lexikon, 9. Auflage, Bd. 1 auf Seite 623 mit Literaturhinweisen beschrieben.

Als direktziehende Haarfarbstoffe für die Haartönungsmittel werden beispielhaft 4-Hydroxypropylamino-3-nitrophenol, HC Red No. 3, 4-Amino-3-nitrophenol, 2-Amino-6-chlor-4-nitrophenol, HC Blue No. 2, HC Blue No. 12, HC Yellow No. 2 und HC Yellow No. 12 genannt, die einzeln oder im Gemisch auch mit weiteren und/oder anderen dem Fachmann bekannten direktziehenden Haarfarbstoffen für den gleichen Zweck enthalten sein können.

Als Beispiele für einzusetzende Entwicklerkomponenten (Oxidationsfarbstoffvorprodukte) sind primäre aromatische oder heteroaromatische Amine mit einer weiteren in p-Stellung befindlichen funktionellen Gruppe, wie p-Phenylendiamin, p-Toluylendiamin, p-Aminophenol, 4-Amino-m-cresol, 2-Hydroxyethyl-1,4-diaminobenzol, 2,4,5,6-Tetraaminopyrimidin, 4,4'-Diaminodiphenylamin und ihre Derivate, weitere Verbindungen der genannten Art, die zusätzlich eine oder mehrere funktionelle Gruppen, wie OH-Gruppen, NH₂-Gruppen, NHR-Gruppen, NRR-Gruppen tragen, wobei R einen gegebenenfalls substituierten Alkylrest mit 1 bis 4 Kohlenstoffatomen darstellt, anzuführen. Ferner können weitere, dem Fachmann bekannte, Entwicklerkomponenten einzeln oder im Gemisch Verwendung finden.

Kupplerkomponenten (Oxidationsfarbstoffvorprodukte), wie z.B. α-Naphthol, Resorcin, 4-Chlorresorcin, 2-Methylresorcin, m-Aminophenol, o-Aminophenol, m-Phenylendiamin, 1,5-bzw. 2,7-Dihydroxynaphthalin, 4-Amino-2-hydroxytoluol, 2,4-Diaminophenoxyethanol, 3-Amino-2,4-dichlorphenol, 2-Amino-4-hydroxyethylaminoanisol, 2-Amino-3-hydroxypyridin, 2,6-Diaminopyridin bzw. Derivate der genannten Verbindungen, können eingesetzt werden. Auch weitere, dem Fachmann bekannte Kupplerkomponenten sind einzeln oder im Gemisch einsetzbar.

Darüber hinaus können die Haarfärbemittel auf Oxidationsfarbstoffbasis gegebenenfalls übliche direktaufziehende Haarfarbstoffe, wie z.B. 4-Hydroxypropylamino-3-nitrophenol, HC Red No. 3, 4-Amino-3-nitrophenol, 2-Amino-6-chlor-4-nitrophenol, HC Blue No. 2, HC Yellow No. 2, HC Blue No. 12, HC Yellow No. 12, HC Red No. 13, HC Red No. 3, HC Yellow No. 6, HC Red No. 10, und/oder weitere, dem Fachmann bekannte, direktziehende Farbstoffe einzeln oder im Gemisch enthalten, falls dies zur Erzielung gewisser Farbnuancen erforderlich ist. Zu den direktziehenden Haarfarbstoffen gehören ebenfalls synthetisch hergestellte oder extrahierte Naturfarbstoffe, wie z.B. Indigo ([2,2'-Biindolin]-3,3'-dion), Maclurin (2,3',4,4',6-Pentahydroxybenzophenon), Brasilin (C.I. Natural Red 24), Hämatoxylin, Alizarin (1,2-Dihydroxyanthrachinon), Juglon (5-Hydroxy-1,4-naphthochinon), Curcumin (2,3,4,6-Tetrahydroxy-5H-benzocyclo-6,8-heptadien-5-on), Carminsäure (C.I. Natural Red 4), oder deren chemisch abgewandelten Derivate.

Bevorzugte Ausführungsformen der Erfindung für die Erzielung der dort angegebenen Farbnuancen sind in der Tabelle II aufgeführt, wobei die bevorzugten Kombinationen von Oxidationsfarbstoffvorprodukten und gegebenenfalls direktziehenden Farbstoffen in einer Menge von ca. 0,1 bis 25 Gew.-% und Serin als Pflegestoff in einer Menge von 0.5 bis 2.5 Gew.-%, bezogen auf die Gesamtmenge des kosmetischen Trägers, enthalten sind. Die in Tabelle II aufgeführten Zahlen geben die Gew.-%-Bereiche der Oxidationsfarbstoffvorprodukte bzw. Direktzieher wieder, die mit folgenden Abkürzungen codiert sind:
Entwickler:
   - A-1: p-Toluylendiamin
   - A-2: 4-Amino-m-cresol
Kuppler:
   - B-1: Resorcin
   - B-2: α-Naphthol
   - B-3: 2-Methylresorcin
   - B-4: m-Aminophenol
   - B-5: 4-Chlorresorcin
   - B-6: 4-Amino-2-hydroxytoluol
   - B-7: 2-Amino-4-hydroxyethylamino-anisol
   - B-8: 2-Amino-3-hydroxypyridin
Direktzieher:
   - C-1: 4-Hydroxypropylamino-2-nitro-phenol
   - C-2: HC Red No. 3
   - C-3: 4-Amino-3-nitrophenol
   - C-4: 2-Amino-6-chlor-4-nitrophenol

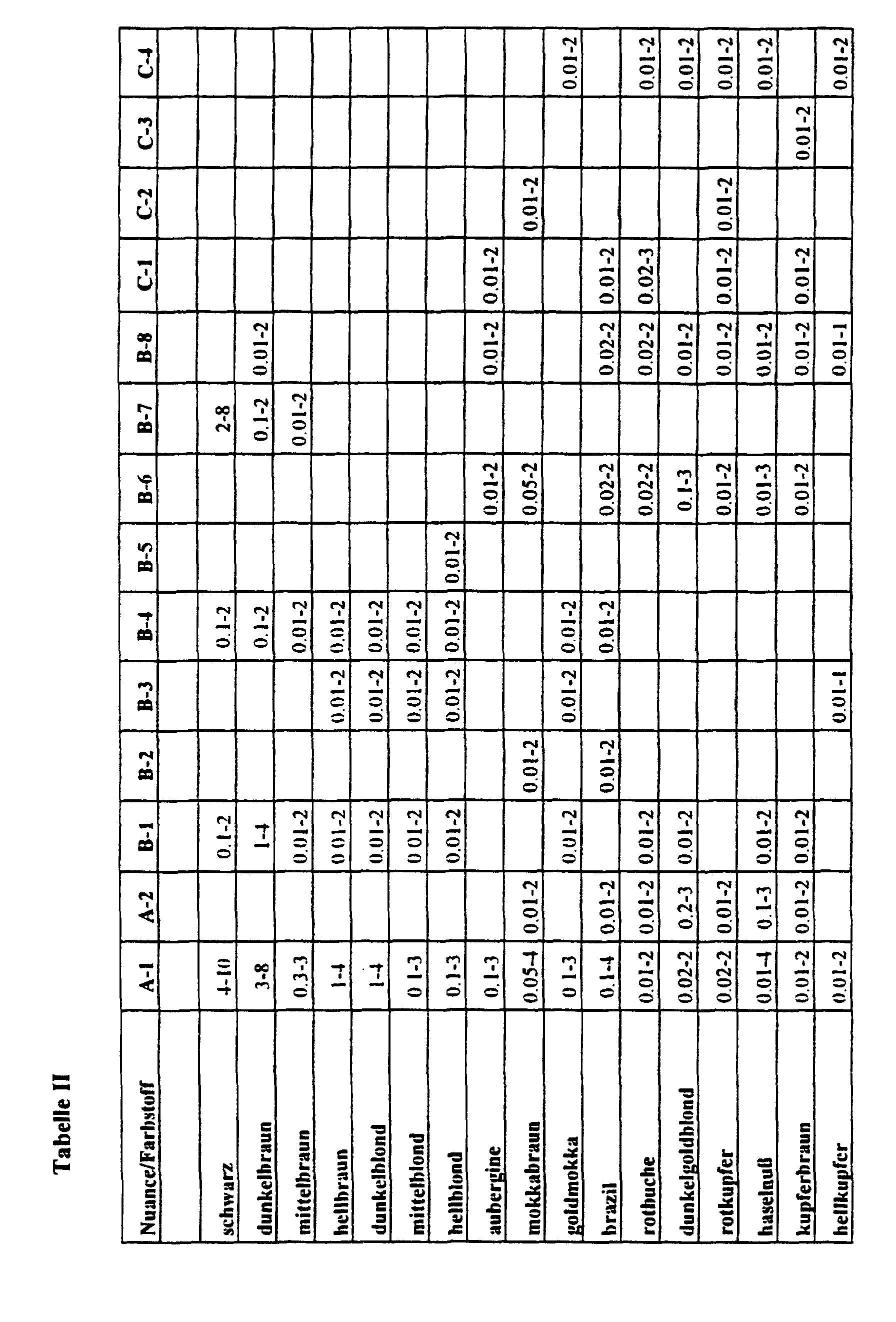

Die Zusammensetzungen der Haarfärbemittel stellen eine Mischung der Farbstoffkomponenten mit den für solche kosmetischen Zubereitungen üblichen Zusätzen dar.

Übliche Zusätze in Lösungen, Cremes, Emulsionen, Gelen oder Schaumaerosolen sind z.B. Lösungsmittel wie Wasser, niedere aliphatische Alkohole, beispielsweise Ethanol, Propanol und Isopropanol, Glykole, Glycerin und Glykolether wie Propylenglykol, weitere Netzmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren.

Die erwähnten Bestandteile werden in den für solche Zwecke üblichen Mengen verwendet, zum Beispiel die Netzmittel und Emulgatoren in Konzentrationen von etwa 0.5 bis 30 Gew.-%, während die Verdicker in einer Menge von etwa 0.1 bis 25 Gew.-% in den Zubereitungen enthalten sein können.

Je nach Zusammensetzung können die erfindungsgemäßen Haarfärbemittel schwach sauer, neutral oder alkalisch reagieren. Insbesondere weisen sie einen pH-Wert im Bereich zwischen 6 und 12 auf, wobei die Einstellung mit Ammoniak, organischen Aminen, wie Monoethanolamin und Triethanolamin oder auch anorganischen Basen, wie Natrium- und Kaliumhydroxyd, erfolgen kann. Die bevorzugte Variante des Haarfärbemittels weist vor Zusatz von wäßrigen Oxidationsmittel-Lösungen einen pH-Bereich zwischen 7 und 8 auf, während es nach 1:1 (w/w) Vermischung mit einer wäßrigen Oxidationsmittel-Lösung als gebrauchsfertiges Haarfärbegel einen pH-Wert von 6.5 bis 7.2 aufweist.

Bei Verfahren zur oxidativen Färbung von Haaren vermischt man die Färbemittel dieser Erfindung, welche eine Kombination von mindestens einer in der Haarfärbung bekannten Entwicklersubstanz mit mindestens einer Kupplersubstanz sowie den Pflegestoffen gemäß Erfindung, nämlich 0.01 Gew.-% bis 10 Gew.-% Verbindungen der allgemeinen Formel (I) und gegebenenfalls einen oder mehrere weitere Pflegestoffe, und gegebenenfalls zusätzlich direktaufziehende Farbstoffe enthalten, kurz vor dem Gebrauch mit einem Oxidationsmittel und trägt dieses Gemisch auf das Haar auf Als Oxidationsmittel zur Entwicklung der Haarfärbung kommen hauptsächlich Wasserstoffperoxid, beispielsweise als 6%ige wäßrige Lösung und dessen Additionsverbindungen an Harnstoff, Melamin oder Natriumborat sowie Gemische aus derartigen Wasserstoffperoxidanlagerungsverbindungen mit Kaliumperoxodisulfat in Betracht. Die oxidative Entwicklung der Färbung kann aber grundsätzlich auch mit Luftsauerstoff erfolgen. Die Anwendungstemperaturen bewegen sich dabei im Bereich von 15 bis 40 °C. Nach einer Einwirkdauer von ca. 30 Minuten wird das Haarfärbemittel vom gefärbten Haar durch Spülen entfernt. Hiernach kann das Haar mit einem Shampoo nachgewaschen werden.

Durch den/die im Haarfärbemittel der Erfindung enthaltenen Pflegestoff(e) gemäß der allgemeinen Formel (I) gemäß Anspruch 1 werden dem Haar schon während und nach dem Färbevorgang neben verbesserter Kämmbarkeit, verbessertem Griff, erhöhtem Glanz und verbessertem Farbausgleich zusätzlich überraschend vorteilhafte Eigenschaften in Form von einer meßbaren, bleibenden Strukturstabilisierung verliehen.

Die vorteilhaften Eigenschaften der mit dem Haarfärbemittel der Erfindung gefärbten Humanhaare wurden durch die folgenden Prüfungen ermittelt:
*1. HP-DSC (High Pressure Differential Scanning Calorimetry)*
   Thermoanalytische Untersuchungen eignen sich besonders zur Charakterisierung von Zweiphasensystemen, zu denen die Humanhaare als Faserkeratine mit ihrem kristallinen α-Helix-Anteil und amorphen Matrix-Anteil ebenfalls gehören. Auf der einen Seite können Glasübergänge und Alterungsverhalten der amorphen Matrix untersucht werden, auf der anderen Seite liefert das Schmelzverhalten der kristallinen, helicalen Phase wichtige Erkenntnisse. Thermoanalytische Untersuchungen sind erstmals 1899 beschrieben (W.C. Roberts-Austen, Proc. Inst. Mech. Eng., London 1899, Seite 35ff.), erste Differenzthermoanalysen (DTA) an Proteinfasern wurden Ende der fünfziger Jahre durchgeführt (F. Schwenker, J.H. Dusenbury, Text. Res. J 1963, 30, Seite 800ff., W.D. Felix, M.A. Mcdowall, H. Eyring, ibid. 1963, 33, Seite 465ff.). In den folgenden Jahren sind unterschiedliche thermoanalytische Meßverfahren, wie DTA, HP-DTA (High Pressure, Hochdruck-DTA) und DSC (Differential Scanning Calorimetry, Dynamische Differenz-Kalorimetrie), an Keratinfasern angewendet worden (G. Ebert, F.H. Müller, Proc. Int. Woll Text. Res. Conf., Cirtel, Paris 1965, 4, Seite 487ff.; G. Ebert, J. Wendorff, Kolloid-Zeitschrfit und Zeitschrift für Polymere 1967, 216/217, Seite 277ff., M. Spei, R. Holzem, Colloid and Polymer Sci. 1987, 265, Seite 965ff.; M. Spei, R. Holzem, Mell. Text. 1987, 68, Seite 923ff.; A.R. Haly, J.W. Snaith, Text. Res. J. 1967, 37, Seite 898ff.; J.S. Crighton, E.R. Hole, Proc. 7th Int. Wool Text. Res. Conf. 1985, Vol. I, Seite 283; J.S. Crighton, W.M. Findon, Proc. 6th Int. Wool Text. Res. Conf., Pretoria 1980, Vol. V, seite 235ff.), um z.B. das Phänomen der Superkontaktion, α-β-Phasenübergange der Helices oder Denaturierungsvorgänge zu untersuchen. In jüngster Zeit wird, insbesondere am Deutschen Wollforschungsinstitut in Aachen (F.J. Wortmann, H. Deutz, J. Appl. Polym. Sci. 1993, 48, Seite 137ff.), die Methode der HP-DSC zur Untersuchung von Keratinfasern genutzt, die die Probleme mit pyrolytischen Effekten, wie sie bei der konventionellen DSC auftreten, und Probleme mit der Datenerfassung und -interpretation, wie sie die DTA birgt, ausschließt. Dabei werden DSC-Messungen an Keratinen durchgeführt, die mit Wasser in kommerziell erhältlichen, druckfesten Meßkapseln eingeschlossen sind. Im Keratin-Wasser-System entwickelt sich beim Erhitzen oberhalb von 100 °C in der verkapselten Stahltiegeln ein Wasserdampfhochdruck, woraus sich die HP-DSC Analyse ableitet. Der gravierende Unterschied der HP-DSC-Thermogramme von Humanhaaren im Vergleich zu normalen DSC-Thermogrammen ist der, daß die endothermen Peaks, die den Umwandlungspunkt und Umwandlungsenthalpie wiedergeben, hier um ca. 90 °C zu niedrigeren Temperaturen verschoben sind. Das rührt daher, daß das Wasser nach Diffusion in die Haarfaser durch Schwächung und Spaltung von Wasserstoffbrücken- und Salzbindungen die Proteinstabilität vermindert und so die "Verleimungstemperatur" der Keratine herabsetzt. Werden durch das superkontrahierende Agens wie Wasser nur Wasserstoffbrücken und Salzbrücken gelöst, so ist der thermische Effekt reversibel (Superkontaktion). Der Vorgang wird jedoch irreversibel, sobald auch kovalente Bindungen, wie z.B. Disulfidbrücken, gespalten werden. Dies tritt ein, wenn man Humanhaarfasern in druckfesten Kapseln mit Wasser auf über 150 °C erhitzt. Die irreversible Umwandlung, interpretiert als Übergang der α-helicalen Bereiche in den Proteinen in einen ungeordneten Zustand, resultiert in endothermen Peaks, wobei die Peaklage den Umwandlungs- oder auch Schmelzpunkt und die Peakfläche die Umwandlungs- oder Schmelzenthalpie wiedergibt.
   Unter Verwendung der Dynamischen Differenz-Kalorimetrie (DSC) können demnach strukturelle sowie chemische Zustände und Veränderungen in Faserkeratinen und insbesondere in Humanhaaren erfaßt werden. Unter genau definierten Versuchsbedingungen kann man bei Humanhaaren die kalorimetrisch erfaßbaren Vorgänge anhand von Thermogrammen aufzeichnen und sie bezüglich der Peaklagen, -strukturen und -flächen als Indikator für die Beeinflussung von Ordnungs-Unordnungsübergangen durch Änderung innerer und/oder äußerer Parameter, hervorgerufen z.B. durch kosmetische Behandlung der Haare, verwenden. D.h. aus den im Thermogramm von Humanhaaren aufgezeichneten endothermen Peaks lassen sich aufgrund von Peaklage (Umwandlungspunkt) und Peakfläche (Umwandlungsenthalpie) Aussagen über Festigkeit bzw. Schädigung der Humanhaarfaser treffen.
   Ausführliche Untersuchungen bezüglich des Einflusses des Cystingehaltes auf die Denaturierung der α-Helices in Keratinen haben z.B. gezeigt, daß die Schmelztemperatur (Übergangstemperatur) des Keratins linear mit dem Cystingehalt ansteigt. Die erhöhte Stabilität des Matrixbereichs aufgrund des höheren Vernetzungsgrades des erhöhten Anteils an Disulfidbrücken in der Matrix führt dazu, daß Umwandlung der in diese Matrix eingebetteten Helices erschwert wird und resultiert somit in einer Schmelztemperatur-Erhöhung. Umgekehrt kann in der Regel eine Schmelzpunkts- und vor allem Schmelzenthalpieerniedrigung bei durch Dauerwelle oder Bleichung bzw. Färbung behandelten Humanhaaren beobachtet werden (H. Deutz, Doktorarbeit, RWTH Aachen 1993).
   Der/die in den erfindungsgemäßen Haarfärbemitteln enthaltene(n) Pflegestoff(e), wie z.B. Serin, dringen in das Haar ein und bilden aufgrund ihres polaren Charakters zusätzliche Wasserstoffbrücken- und Salzbindungen in der Matrix aus, was wiederum zu einer erhöhten Stabilität gegenüber Deformierung führt und somit eine generelle Strukturstabilisierung des Haares bewirkt. Dies wird in thermoanalytischen Untersuchungen an den mit erfindungsgemäßen Färbemitteln behandelten Humanhaaren durch eine Verschiebung des Umwandlungs- bzw. Schmelzpunktes der Humanhaarfasern zu höheren Temperaturen hin ersichtlich.
2. Darüber hinaus wird die durch die erfindungsgemäßen Haarfärbemittel hervorgerufene Strukturstabilisierung durch dem Fachmann bekannte Zug-Dehnungs-Messungen an den entsprechend gefärbten Humanhaaren belegt.
3. Weiterhin wird die durch die erfindungsgemäßen Haarfarbemittel hervorgerufene Strukturstabilisierung durch den sogenannten Ermüdungstest nachgeweisen. Dabei werden die zu untersuchenden Humanhaare nach Konditionierung bei 21 °C und 65 % RH einzeln am oberen Ende an einer Plattform eingespannt und am unteren Ende mit einem Gewicht (30 bis 40 g) versehen, so daß es gerade am Boden aufliegt. Die Plattform hebt sich in vertikaler Richtung auf und nieder, so daß die Haarfasern abwechselnd be- und entlastet werden. Es werden normalerweise 100 000 Cyclen mit einer Frequenz von I Cyclus/Sekunde durchgeführt und die Überlebensrate ermittelt. Aus dieser Überlebensrate wird die Stabilität der Fasern relativ zueinander ermittelt.

Die Erfindung wird durch folgende Beispiele verdeutlicht :

### Beispiel 1

| ***Oxidationshaarfarbe in Cremeform*** | |
|---|---|
| p-Toluylendiamin | 1,85 g |
| m-Aminophenol | 0,25 g |
| Resorcin | 0,65 g |
| 2-Amino-4-(2-hydroxyethyl)-aminoanisol | 0,05 g |
| Laurylalkoholdiglykolethersulfat, Natriumsalz (28%ige Lsg.) | 5,00 g |
| Cetylalkohol | 10,00 g |
| Myristylalkohol | 6,00 g |
| Ölsäure | 1,00 g |
| Ammoniak, 25%ig | 8,00 g |
| Natriumdithionit | 0,40 g |
| L-Serin | 2,00 g |
| Wasser add | 100,00 g |

50 g der vorstehend genannten Creme wird als Haarfärbemittel kurz vor dem Gebrauch mit 50 g Wasserstoffperoxidlösung (6 Gew.-%) gemischt. Man läßt das Gemisch 30 Minuten bei Raumtemperatur auf 50 % ergraute Naturhaare einwirken. Danach wird das Haarfärbemittel ausgespült, das Haar shampooniert und getrocknet. Es hat einen gleichmäßigen, farbsatten mittelbraunen Ton erhalten.

Daß die im Haarfärbemittel nach Beispiel 1 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden strapazierte Humanhaarsträhnen A und B wie folgt behandelt:
A: Färbung mit einer 1:1 Mischung aus Färbecreme nach Beispiel 1 und Wasserstoffperoxidlösung (6 Gew.-%) für 30 Minuten bei Raumtemperatur und anschließendes Ausspülen und Trocknen
B. Färbung mit einer 1:1 Mischung aus Färbecreme nach Beispiel 1 und Wasserstoffperoxidlösung (6 Gew.-%) für 30 Minuten bei Raumtemperatur, jedoch ohne Zusatz der Pflegestoffe gemäß Anspruch 1 bis 28, in diesem Beispiel L-Serin, und anschließendes Ausspülen und Trocknen

An den, wie oben ausgeführt, gefärbten Humanhaarsträhnen A und B wurden thermoanalytische Untersuchungen vorgenommen. Diese ergaben für Strähne A gegenüber Strähne B einen signifikant erhöhten Schmelzpunkt.

### Beispiel 2

| ***Haartönungsmittel*** | |
|---|---|
| HC Red No. 3 | 0,75 g |
| HC Yellow No. 2 | 0,20 g |
| 4-(3-Hydroxypropylamino)-3-nitrophenol | 0,20 g |
| Hydroxyethylcellulose | 0,90 g |
| Monoäthanolaminlaurylsulfat | 5,00 g |
| Cetylalkohol | 1,00 g |
| D/L-Serin | 1,00 g |
| L-Tyrosin | 1,00 g |
| Ethyldiglykol | 5,00 g |
| Wasser add. | 100,00 g |

Dunkelblondes Naturhaar wird 30 Minuten bei 35°C mit der Lösung aus Beispiel 2 behandelt, danach mit Wasser gespült und anschließend getrocknet. Das Haar hat einen intensiven leuchtenden Rotton erhalten.

Daß die im Haarfärbemittel nach Beispiel 2 enthaltenen, erfindungsgemäßen Pflegestoffe im Haar schon während des Tönungsvorganges eine deutliche und bleibende Strukturstabilisierung bewirken, geht aus folgenden Untersuchungen hervor:

Es wurden strapazierte, dunkelblonde Humanhaarsträhnen A und B wie folgt behandelt:
A: Tönung mit der in Beispiel 2 aufgeführten Lösung für 30 Minuten bei 35 °C und anschließendes Ausspülen und Trocknen
B: Tönung mit der in Beispiel 2 aufgeführten Lösung für 30 Minuten bei 35 °C, jedoch ohne Zusatz der Pflegestoffe gemäß Anspruch 1 bis 28, in diesem Beispiel D/L-Serin und L-Tyrosin, und anschließendes Ausspülen und Trocknen

An den, wie oben ausgeführt, getönten Humanhaarsträhnen A und B wurden sowohl thermoanalytische Untersuchungen als auch Zug-Dehnungs-Messungen vorgenommen.

Die thermoanalytischen Untersuchungen ergaben für Strähne A gegenüber Strähne B einen signifikant erhöhten Schmelzpunkt. Die Zug-Dehnungs-Messungen ergaben für Strähne A gegenüber Strähne B eine signifikant verbesserte Festigkeit.

### Beispiel 3

| ***Oxidationshaarfarbe in Cremeform*** | |
|---|---|
| p-Phenylendiamin | 0,75 g |
| Resorcin | 0,28 g |
| o-Aminophenol | 0,01 g |
| m-Aminophenol | 0,07 g |
| 2,6-Diaminopyridin | 0,02 g |
| p-Amino-o-cresol | 0,01 g |
| m-Phenylendiamin | 0,01 g |
| 1,2-Propylenglycol | 10,00 g |
| Polyethylenglycolcocosamin | 7,00 g |
| Isopropanol | 10,00 g |
| Cocamide DEA | 20,00 g |
| Ölsäure | 8,00 g |
| Natrimsulfit | 0,20 g |
| EDTA | 0,20 g |
| Ammoniak, 25 %ig | 6,00 g |
| D/L-Serin | 1,00 g |
| L-Lysin | 1,00 g |
| Wasser add. | 100,00 g |

50 g der vorstehend genannten Creme werden kurz vor Gebrauch mit 50 g Wasserstoffperoxidlösung (6 Gew.-%) gemischt. Man läßt das Gemisch 30 Minuten bei Raumtemperatur auf 50 % ergrautes Haar einwirken. Anschließend wird das Färbemittel ausgespült und das Haar shampooniert und getrocknet. Es hat einen gleichmäßigen hellaschbraunen Farbton erhalten.

Daß die im Haarfärbemittel nach Beispiel 3 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden strapazierte Humanhaarsträhnen A und B wie folgt behandelt:
A. Färbung mit einer 1:1 Mischung aus Färbecreme nach Beispiel 3 und Wasserstoffperoxidlösung (6 Gew.-%) für 30 Minuten bei Raumtemperatur und anschließendes Ausspülen und Trocknen
B: Färbung mit einer 1:1 Mischung aus Färbecreme nach Beispiel 1 und Wasserstoffperoxidlösung (6 Gew.-%) für 30 Minuten bei Raumtemperatur, jedoch ohne Zusatz der Pflegestoffe gemäß Anspruch 1 bis 28, in diesem Beispiel D/L-Serin und L-Lysin, und anschließendes Ausspülen und Trocknen

An den, wie oben ausgeführt, gefärbten Humanhaarsträhnen A und B wurden sowohl thermoanalytische Untersuchungen als auch Zug-Dehnungs-Messungen vorgenommen.

Die thermoanalytischen Untersuchungen ergaben für Strähne A gegenüber Strähne B einen signifikant erhöhten Schmelzpunkt. Die Zug-Dehnungs-Messungen ergaben für Strähne A gegenüber Strähne B eine signifikant verbesserte Festigkeit.

### Beispiel 4

| ***Oxidationshaarfarbe in Gelform*** | |
|---|---|
| p-Toluylendiamin | 0,20 g |
| 4-Amino-m-cresol | 1,00 g |
| Resorcin | 0,09 g |
| 4-Amino-2-hydroxytoluol | 1,00 g |
| 2-Amino-3-hydroxypyridin | 0,10 g |
| 2-Amino-6-chlor-4-nitrophenol | 0,05 g |
| Polyacrylsäure, Ammoniumsalz, 10 %ig | 15,00 g |
| Cetylstearylalkohol | 8,00 g |
| Cetylstearylsulfat, Natriumsalz | 1,00 g |
| PEG 40 Rizinusöl | 1,00 g |
| Dinatriumlaureth-5-sulfosuccinat (Lanolinsulfosuccinat) | 4,00 g |
| Natriumsulfit | 0,20 g |
| Monoethanolamin | 0,30 g |
| L-Lysin | 2,00 g |
| Wasser add. | 100,00 g |

50 g des vorstehend genannten Gels werden kurz vor der Anwendung mit 50 g Wasserstoffperoxidlösung (3 Gew.-%) vermischt und gleichmäßig auf mittelblondes Naturhaar aufgetragen. Nach einer Einwirkzeit von 30 Minuten bei Raumtemperatur wird das Haarfärbegel ausgespült, das Haar shampooniert und getrocknet. Es hat einen gleichmäßigen haselnußbraunen Farbton erhalten.

Daß die im Haarfärbemittel nach Beispiel 4 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden strapazierte Humanhaarsträhnen A und B wie folgt behandelt:
A. Färbung mit einer 1:1 Mischung aus Färbegel nach Beispiel 4 und Wasserstoffperoxidlösung (3 Gew.-%) für 30 Minuten bei Raumtemperatur und anschließendes Ausspülen und Trocknen
B: Färbung mit einer 1:1 Mischung aus Färbegel nach Beispiel 4 und Wasserstoffperoxidlösung (3 Gew.-%) für 30 Minuten bei Raumtemperatur, jedoch ohne Zusatz der Pflegestoffe gemäß Anspruch 1 bis 28, in diesem Beispiel L-Lysin, und anschließendes Ausspülen und Trocknen

An den, wie oben ausgeführt, gefärbten Humanhaarsträhnen A und B wurden thermoanalytische Untersuchungen durchgeführt. Diese ergaben für Strähne A gegenüber Strähne B einen signifikant erhöhten Schmelzpunkt.

### Beispiel 5

| ***Oxidationshaarfarbe in Cremeform*** | |
|---|---|
| p-Toluylendiamin | 0,50 g |
| Resorcin | 0,15 g |
| 2-Methylresorcin | 0,06 g |
| m-Aminophenol | 0,50 g |
| Polyacrylsäure, Ammoniumsalz, 1 %ig | 17,00 g |
| Cocamidopropyl Betain | 18,00 g |
| EDTA | 0,30 g |
| Cetylalkohol | 2,00 g |
| Fettalkoholpolyglycolether | 7,00 g |
| Myristylalkohol | 1,50 g |
| KOH, 50 %ig | 0,50 g |
| Natriumdithionit | 0,30 g |
| D/L-Serin | 0,50 g |
| L-Threonin | 0,50 g |
| Wasser add. | 100,00 g |

35 g des vorstehend genannten Gels werden kurz vor der Anwendung mit 70 g Wasserstoffperoxidlösung (1.5 Gew.-%) vermischt und nach der Auftragung 30 Minuten bei Raumtemperatur auf dem Haar belassen. Anschließend wird das Haarfärbegel ausgespült, das Haar shampooniert und getrocknet. Es hat einen mittelblonden Farbton erhalten.

Daß die im Haarfärbemittel nach Beispiel 5 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden leicht strapazierte Humanhaarsträhnen A und B wie folgt behandelt:
A: Färbung mit einer 1:2 Mischung aus Färbegel nach Beispiel 5 und Wasserstoffperoxidlösung (1.5 Gew.-%) für 30 Minuten bei Raumtemperatur und anschließendes Ausspülen und Trocknen
B: Färbung mit einer 1:2 Mischung aus Färbegel nach Beispiel 5 und Wasserstoffperoxidlösung (1.5 Gew.-%) für 30 Minuten bei Raumtemperatur, jedoch ohne Zusatz der Pflegestoffe gemäß Anspruch 1 bis 28, in diesem Beispiel D/L-Serin und L-Threonin, und anschließendes Ausspülen und Trocknen

An den, wie oben ausgeführt, gefärbten Humanhaarsträhnen A und B wurden sowohl thermoanalytische Untersuchungen als auch Zug-Dehnungs-Messungen vorgenommen.

Die thermoanalytischen Untersuchungen ergaben für Strähne A gegenüber Strähne B einen signifikant erhöhten Schmelzpunkt. Die Zug-Dehnungs-Messungen ergaben für Strähne A gegenüber Strähne B eine signifikant verbesserte Festigkeit.

### Beispiel 6

| ***Oxidationshaarfarbe in Gelform*** | |
|---|---|
| p-Toluylendiamin | 0,85 g |
| Resorcin | 0,30 g |
| 2-Methylresorcin | 0,08 g |
| m-Aminophenol | 0,07 g |
| Polyacrylsäure, Ammoniumsalz, 10 %ig | 15,00 g |
| Cetylstearylalkohol | 8,00 g |
| Cetylstearylsulfat, Natriumsalz | 1,00 g |
| PEG 40 Rizinusöl | 1,00 g |
| Dinatriumlaureth-5-sulfosuccinat (Lanolinsulfosuccinat) | 4,00 g |
| Natriumsulfit | 0,20 g |
| Monoethanolamin | 0,30 g |
| D/L-Serin | 2,00 g |
| Wasser add. | 100,00 g |

50 g des vorstehend genannten Gels werden kurz vor der Anwendung mit 50 g Wasserstoffperoxidlösung (1.5 Gew.-%) vermischt und gleichmäßig auf 50 % ergrautes Naturhaar aufgetragen. Nach einer Einwirkzeit von 30 Minuten bei Raumtemperatur wird das Haarfärbegel ausgespült, das Haar shampooniert und getrocknet. Es hat einen gleichmäßigen dunkelblonden Farbton erhalten.

Daß die im Haarfärbemittel nach Beispiel 6 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden leicht strapazierte Humanhaarsträhnen A und B wie folgt behandelt:
A: Färbung mit einer 1:1 Mischung aus Färbegel nach Beispiel 6 und Wasserstoffperoxidlösung (1.5 Gew.-%) für 30 Minuten bei Raumtemperatur und anschließendes Ausspülen und Trocknen
B: Färbung mit einer 1:1 Mischung aus Färbegel nach Beispiel 6 und Wasserstoffperoxidlösung (1.5 Gew.-%) für 30 Minuten bei Raumtemperatur, jedoch ohne Zusatz der Pflegestoffe gemäß Anspruch 1 bis 28, in diesem Beispiel D/L-Serin, und anschließendes Ausspülen und Trocknen

An den, wie oben ausgeführt, gefärbten Humanhaarsträhnen A und B wurden thermoanalytische Untersuchungen, Zug-Dehnungs-Messungen und Ermüdungstests durchgeführt.

Die thermoanalytischen Untersuchungen ergaben für Strähne A gegenüber Strähne B einen signifikant erhöhten Schmelzpunkt. Die Zug-Dehnungs-Messungen ergaben für Strähne A gegenüber Strähne B eine signifikant verbesserte Festigkeit. Der Ermüdungstest zeigte für Strähne A gegenüber Strähne B eine signifikant höhere Überlebensrate.

### Beispiel 7

| ***Oxidationshaarfarbe in Gelform*** | |
|---|---|
| p-Toluylendiamin | 8,55 g |
| Resorcin | 1,00 g |
| m-Aminophenol | 0.20 g |
| 2-Amino-4-(2-hydroxyethyl)-aminoanisol | 6,07 g |
| Polyacrylsäure, Ammoniumsalz, 10 %ig | 15,00 g |
| Cetylstearylalkohol | 8,00 g |
| Cetylstearylsulfat, Natriumsalz | 1,00 g |
| PEG 40 Rizinusöl | 1,00 g |
| Dinatriumlaureth-5-sulfosuccinat (Lanolinsulfosuccinat) | 4,00 g |
| Natriumsulfit | 0,20 g |
| Monoethanolamin | 0,30 g |
| L-Serin | 1,00 g |
| L-Tryptophan | 1,00 g |
| Wasser add. | 100,00 g |

50 g des vorstehend genannten Gels werden kurz vor der Anwendung mit 50 g Wasserstoffperoxidlösung (3 Gew.-%) vermischt und gleichmäßig auf 50 % ergrautes Naturhaar aufgetragen. Nach einer Einwirkzeit von 30 Minuten bei Raumtemperatur wird das Haarfärbegel ausgespült, das Haar shampooniert und getrocknet. Es hat einen gleichmäßigen tiefschwarzen Farbton erhalten.

Daß die im Haarfärbemittel nach Beispiel 7 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden strapazierte Humanhaarsträhnen A und B wie folgt behandelt:
A: Färbung mit einer 1:1 Mischung aus Färbegel nach Beispiel 7 und Wasserstoffperoxidlösung (3 Gew.-%) für 30 Minuten bei Raumtemperatur und anschließendes Ausspülen und Trocknen
B: Färbung mit einer 1:1 Mischung aus Färbegel nach Beispiel 7 und Wasserstoffperoxidlösung (3 Gew.-%) für 30 Minuten bei Raumtemperatur, jedoch ohne Zusatz der Pflegestoffe gemäß Anspruch 1 bis 28, in diesem Beispiel L-Serin und L-Tryptophan, und anschließendes Ausspülen und Trocknen

An den, wie oben ausgeführt, gefärbten Humanhaarsträhnen A und B wurden thermoanalytische Untersuchungen vorgenommen. Diese ergaben für Strähne A gegenüber Strähne B einen signifikant erhöhten Schmelzpunkt.

### Beispiel 8

| ***Oxidationshaarfarbe in Cremeform*** | |
|---|---|
| p-Toluylendiamin | 0,18 g |
| 2-Methylresorcin | 0,09 g |
| 2-Amino-3-hydroxypyridin | 0,03 g |
| 2-Amino-6-chlor-4-nitrophenol | 0,10 g |
| Polyacrylsäure, Ammoniumsalz, 1 %ig | 17,00 g |
| Cocamidopropyl Betain | 18,00 g |
| EDTA | 0,30 g |
| Cetylalkohol | 2,00 g |
| Fettalkoholpolyglycolether | 7,00 g |
| Myristylalkohol | 1,50 g |
| KOH, 50 %ig | 0,50 g |
| Natriumdithionit | 0,30 g |
| L-Lysin | 1,00 g |
| L-Threonin | 0,50 g |
| Wasser add. | 100,00 g |

35 g des vorstehend genannten Gels werden kurz vor der Anwendung mit 70 g Wasserstoffperoxidlösung (1.5 Gew-%) vermischt und nach der Auftragung 30 Minuten bei Raumtemperatur auf dem hellblonden, ergrauten Haar belassen. Anschließend wird das Haarfärbegel ausgespült, das Haar shampooniert und getrocknet. Es hat einen hellkupfernen Farbton erhalten.

Daß die im Haarfärbemittel nach Beispiel 8 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden strapazierte Humanhaarsträhnen A und B wie folgt behandelt:
A: Färbung mit einer 1:2 Mischung aus Färbegel nach Beispiel 8 und Wasserstoffperoxidlösung (1.5 Gew-%) für 30 Minuten bei Raumtemperatur und anschließendes Ausspülen und Trocknen
B: Färbung mit einer 1:2 Mischung aus Färbegel nach Beispiel 8 und Wasserstoffperoxidlosung (1.5 Gew-%) für 30 Minuten bei Raumtemperatur, jedoch ohne Zusatz der Pflegestoffe gemäß Anspruch 1 bis 28, in diesem Beispiel L-Lysin und L-Threonin, und anschließendes Ausspülen und Trocknen

An den, wie oben ausgeführt, gefärbten Humanhaarsträhnen A und B wurden thermoanalytische Untersuchungen vorgenommen. Diese ergaben für Strähne A gegenüber Strähne B einen signifikant erhöhten Schmelzpunkt

### Beispiel 9

| ***Oxidationshaarfarbe in Gelform*** | |
|---|---|
| p-Toluylendiamin | 0,91 g |
| 4-Amino-2-hydroxytoluol | 0,40 g |
| 4-Amino-2-hydroxypyridin | 0,08 g |
| 4-Hydroxypropylamino-2-nitrophenol | 0,25 g |
| Polyacrylsäure, Ammoniumsalz, 10 %ig | 15,00 g |
| Cetylstearylalkohol | 8,00 g |
| Cetylstearylsulfat, Natriumsalz | 1,00 g |
| PEG 40 Rizinusöl | 1,00 g |
| Dinatriumlaureth-5-sulfosuccinat (Lanolinsulfosuccinat) | 4,00 g |
| Natriumsulfit | 0,20 g |
| Monoethanolamin | 0,30 g |
| L-Serin | 1,00 g |
| Wasser add. | 100,00 g |

50 g des vorstehend genannten Gels werden kurz vor der Anwendung mit 50 g Wasserstoffperoxidlösung (3 Gew.-%) vermischt und gleichmäßig auf 50 % ergrautes mittelbraunes Naturhaar aufgetragen. Nach einer Einwirkzeit von 30 Minuten bei Raumtemperatur wird das Haarfärbegel ausgespült, das Haar shampooniert und getrocknet. Es hat einen gleichmäßigen auberginefarbenen Ton erhalten.

Daß die im Haarfärbemittel nach Beispiel 9 enthaltenen, erfindungsgemäßen Pflegestoffe im Haar schon während des Färbevorganges eine deutliche und bleibende Strukturstabilisierung bewirken, geht aus folgenden Untersuchungen hervor:

Es wurden strapazierte Humanhaarsträhnen A und B wie folgt behandelt:
A: Färbung mit einer 1:1 Mischung aus Färbegel nach Beispiel 9 und Wasserstoffperoxidlösung (3 Gew.-%) für 30 Minuten bei Raumtemperatur und anschließendes Ausspülen und Trocknen
B: Farbung mit einer 1:1 Mischung aus Färbegel nach Beispiel 9 und Wasserstoffperoxidlösung (3 Gew.-%) für 30 Minuten bei Raumtemperatur, jedoch ohne Zusatz der Pflegestoffe gemäß Anspruch 1 bis 28, in diesem Beispiel L-Serin, und anschließendes Ausspülen und Trocknen

An den, wie oben ausgeführt, gefärbten Humanhaarsträhnen A und B wurden thermoanalytische Untersuchungen vorgenommen. Diese ergaben für Strähne A gegenüber Strähne B einen signifikant erhöhten Schmelzpunkt.

### Beispiel 10

| ***Oxidationshaarfarbe in Gelform*** | |
|---|---|
| p-Toluylendiamin | 0,85 g |
| Resorcin | 0,30 g |
| 2-Methylresorcin | 0,08 g |
| m-Aminophenol | 0,07 g |
| Polyacrylsäure, Ammoniumsalz, 10 %ig | 15,00 g |
| Cetylstearylalkohol | 8,00 g |
| Cetylstearylsulfat, Natriumsalz | 1,00 g |
| PEG 40 Rizinusöl | 1,00 g |
| Dinatriumlaureth-5-sulfosuccinat (Lanolinsulfosuccinat) | 4,00 g |
| Natriumsulfit | 0,20 g |
| Monoethanolamin | 0,30 g |
| L-Serin | 1,00 g |
| L-Histidin | 0,50 g |
| Wasser add. | 100,00 g |

50 g des vorstehend genannten Gels werden kurz vor der Anwendung mit 50 g Wasserstoffperoxidlösung (1.5 Gew.-%) vermischt und gleichmäßig auf 50 % ergrautes Naturhaar aufgetragen. Nach einer Einwirkzeit von 30 Minuten bei Raumtemperatur wird das Haarfärbegel ausgespült, das Haar shampooniert und getrocknet. Es hat einen gleichmäßigen dunkelblonden Farbton erhalten.

Daß die im Haarfärbemittel nach Beispiel 10 enthaltenen Wirksubstanzen im Haar schon während des Farbevorganges eine deutliche und bleibende Strukturstabilisierung bewirken, geht aus folgenden Untersuchungen hervor:

Es wurden strapazierte Humanhaarsträhnen A und B wie folgt behandelt:
A: Färbung mit einer 1:1 Mischung aus Färbegel nach Beispiel 10 und Wasserstoffperoxidlösung (1.5 Gew.-%) für 30 Minuten bei Raumtemperatur und anschließendes Ausspülen und Trocknen
B: Färbung mit einer 1:1 Mischung aus Färbegel nach Beispiel 10 und Wasserstoffperoxidlösung (1.5 Gew.-%) für 30 Minuten bei Raumtemperatur, jedoch ohne Zusatz der Pflegestoffe gemäß Anspruch 1 bis 28, in diesem Beispiel L-Serin und L-Histidin, und anschließendes Ausspülen und Trocknen

An den. wie oben ausgeführt, gefärbten Humanhaarsträhnen A und B wurden sowohl thermoanalytische Untersuchungen als auch Zug-Dehnungs-Messungen vorgenommen.

Die thermoanalytischen Untersuchungen ergaben für Strähne A gegenüber Strähne B einen signifikant erhöhten Schmelzpunkt. Die Zug-Dehnungs-Messungen ergaben für Strähne A gegenüber Strähne B eine signifikant verbesserte Festigkeit.

### Beispiele 11 bis 15

Die Beispiele 11 bis 15 entsprechen den Beipielen 1 bis 5, jedoch wurden statt der dort jeweils angegebenen Pflegestoffe in diesen Fällen 1 Gew.-% D/L-Serin, 2 Gew.-% Phospholipid EFA und 2 Gew.-% Panthenol als Pflegestoff-Kombination zugesetzt. Thermoanalytische Untersuchungen an den mit den Färbemitteln der Erfindung, enthaltend Serin, Phospholipid EFA und Panthenol als Pflegestoff-Kombination, im Vergleich zu den mit entsprechenden Färbemitteln ohne diese Pflegestoff-Kombination behandelten Humanhaaren zeigten einen eindeutigen strukturstabilisierenden Effekt für die Färbemittel der Erfindung und darüber hinaus einen stärkeren strukturstabilisierenden Effekt als entsprechende Färbemittel mit jeweils nur einem oder zwei der in der Pflegestoff-Kombination der Erfindung enthaltenen Pflegestoffe.

### Beispiele 16 bis 20

Die Beispiele 16 bis 20 entsprechen den Beispielen 6 bis 10, jedoch wurden statt der dort jeweils angegebenen Pflegestoffe in diesen Fällen 1 Gew.-% Serin, 2 Gew.-% Keratinhydrolysat und 4 Gew.-% Sojalecithin als Pflegestoff-Kombination zugesetzt. Thermoanalytische Untersuchungen an den mit den Färbemitteln der Erfindung, enthaltend Serin, Keratinhydrolysat und Sojalecithin als Pflegestoff-Kombination, im Vergleich zu den mit entsprechenden Färbemitteln ohne diese Pflegestoff-Kombination behandelten Humanhaaren zeigten einen eindeutigen strukturstabilisierenden Effekt für die Färbemittel der Erfindung und darüber hinaus einen stärkeren strukturstabilisierenden Effekt als entsprechende Färbemittel mit jeweils nur einem oder zwei der in der Pflegestoff-Kombination der Erfindung enthaltenen Pflegestoffe.

In einer weiteren Ausführungsform der vorliegenden Erfindung enthält die Entwicklerlotion (Oxidationsmittellösung) Pflegestoffe einzeln oder in Kombination gemäß einem oder mehreren der Ansprüche 1 bis 17 und 26 bis 27, wobei die Pflegestoffe bzw. Pflegestoffkombinationen gleich oder verschieden von denen im Färbemittelträger (Färbecreme, -gel oder -pulver) sind. Diese Ausführungsform wird durch die folgenden Beispiele verdeutlicht.

### Beispiele 21 bis 40

Es wurde gemäß den Beispielen 1 und 3 bis 20 gearbeitet. Jedoch abweichend enthielt die Wasserstoffperoxidlösung (Entwicklerlotion) einen Zusatz einer Kombination aus 1 Gew.-% Serin, 2 Gew.-% Phospholipid EFA und 2 Gew.-% Panthenol. Untersuchungen an den mit den Färbemitteln gemäß Beispiel 21 bis 40 gefärbten Humanhaaren, verglichen mit Humanhaaren, die mit dem entsprechenden Färbemittel ohne Pflegestoffe bzw, Pflegestoffkombination der Erfindung behandelt wurden, zeigen eindeutig eine strukturverbessernde und pflegendere Wirkung.

### Beispiele 41 bis 60

Es wurde gemäß den Beispielen 1 und 3 bis 20 gearbeitet. Jedoch abweichend enthielt die Wasserstoffperoxidlösung (Entwicklerlotion) einen Zusatz einer Kombination aus 1 Gew.-% Serin, 2 Gew.-% Keratinhydrolysat und 4 Gew.-% Sojalecithin. Vergleichende Untersuchungen ergaben für die Färbemittel mit Pflegestoff-Kombination gemäß Erfindung eine eindeutig strukturverbessernde und pflegendere Wirkung.

### Beispiele 61-70

Es wurde gemäß den Beispielen 1 bis 10 gearbeitet, jedoch abweichend wurde anstelle der dort eingesetzten Pflegestoffe 2 Gew.-% Phosphoserin als Pflegestoff zugesetzt. Vergleichende Untersuchungen ergaben für die Haarfärbemittel mit Phosphoserin als Pflegestoff eine deutlich pflegendere und strukturstabilisierendere Wirkung gegenüber den entsprechenden Haarfärbemittein ohne Phosphoserin.

### Beispiele 71-80

Es wurde gemäß den Beispielen 1 bis 10 gearbeitet, jedoch abweichend wurde anstelle der dort eingesetzten Pflegestoffe 2 Gew.-% Serinmethylester als Pflegestoff zugesetzt. Vergleichende Untersuchungen ergaben für die Haarfärbemittel mit Serinmethylester als Pflegestoff eine deutlich pflegendere und strukturstabilisierendere Wirkung gegenüber den entsprechenden Haarfärbemitteln ohne Serinmethylester

### Vergleichsmessungen zum Nachweis der Erfindungshöhe

Zum Nachweis der Erfindungshöhe sind im folgenden einige vergleichende Untersuchungen exemplarisch aufgeführt. Als Meßmethode wurde das auf den Seiten 8 bis 10 der allgemeinen Beschreibung erläuterte Verfahren der Thermischen Differenz-Kalorimetrie (DSC) angewandt.

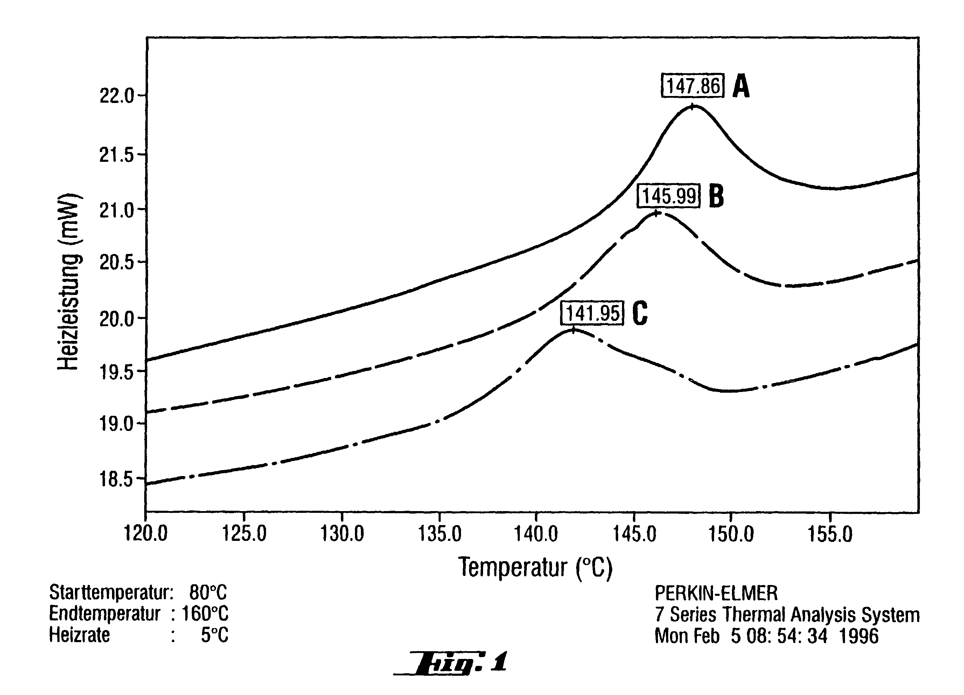

Fig. 1 zeigt den entscheidenden Ausschritt (Schmelzpeak) des Thermogramms einer unbehandelten, strapazierten Humanhaarsträhne (Kurve A) im Vergleich zu den Thermogramm-Ausschnitten der gleichen strapazierten Humanhaarprobe, jedoch eingefärbt mit dem Haarfärbemittel in Cremeform ohne Zusatz eines Pflegestoffes (Kurve C) bzw. mit einem Haarfärbemittel in Cremeform mit 2 Gew.-% L-Serin als Pflegestoff (Kurve B). Die angegebenen Peakmaxima entsprechen dem jeweiligen Schmelzpunkt des Keratins, der direkt mit der Strukturstabilität der Haares korreliert. Man sieht eindeutig, daß durch Färbung des strapazierten Humanhaares mit dem Haarfärbemittel ohne erfindungsgemäßem Pflegestoff eine Schmelzpunkterniedrigung von ursprünglich 148 °C auf 142 °C erfolgt, während durch Färbung des strapazierten Humanhaares mit dem Haarfärbemittel gemäß Erfindung mit 2 Gew.-% L-Serin als Pflegestoff eine wesentlich geringere Schmelzpunkterniedrigung auf 146 °C erfolgt. Durch Zusatz des erfindungsgemäßen Pflegestoffes im Haarfärbemittel wird demnach während des Färbevorganges eindeutig eine geringere Schädigung des Haares hervorgerufen.

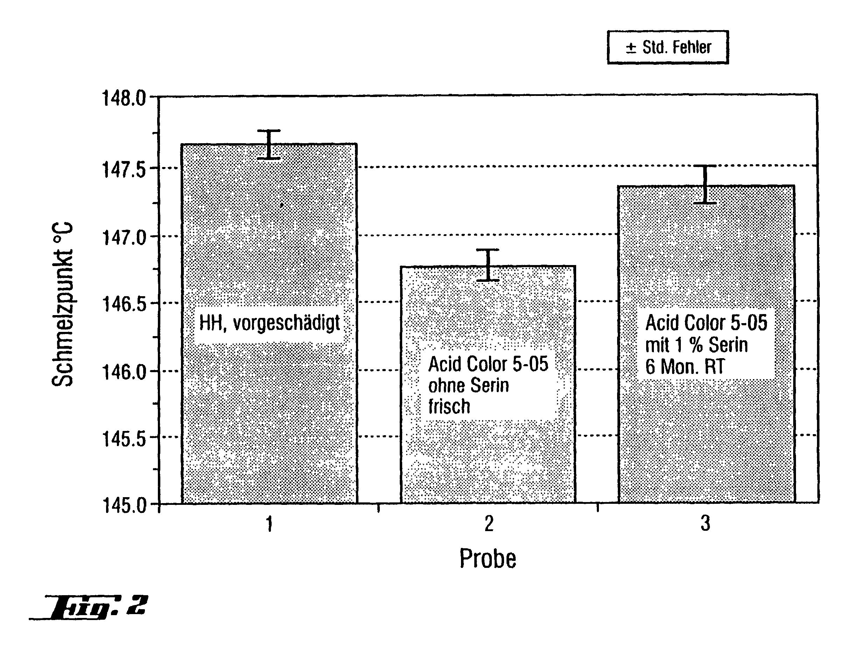

Fig 2 zeigt die statistische Auswertung von vergleichenden thermoanalytischen Untersuchungen an strapazierten Humanhaaren, hier als "vorgeschädigt" bezeichnet (Probe 1), die einerseits mit einem Haarfärbemittel in Gelform ohne Pflegestoff gemäß Erfindung (Probe 2), andererseits mit dem entsprechenden Haarfärbemittel mit 1 Gew.% D/L-Serin als erfindungsgemäßen Pflegestoff (Probe 3) behandelt wurden. Aus dem Diagramm ist eindeutig ersichtlich, daß durch Zusatz von 1 Gew.-% D/L-Serin als Pflegestoff in Haarfärbemitteln eine geringere Schmelzpunkterniedrigung beim Färbevorgang an Humanhaaren erfolgt als ohne Zusatz eines entsprechenden Pflegestoffes, das Haar somit wesentlich geringer geschädigt wird. Darüber hinaus sind die Ergebnisse mit einem frisch hergestellten Haarfärbemittel ohne Pflegestoff gegen ein Haarfärbemittel mit 1 Gew.-% D/L-Serin als Pflegestoff, das bereits 6 Monate bei Raumtemperatur gelagert hatte, erzielt worden, was belegt, daß der Pflegestoff in der Rezeptur absolut stabil ist.

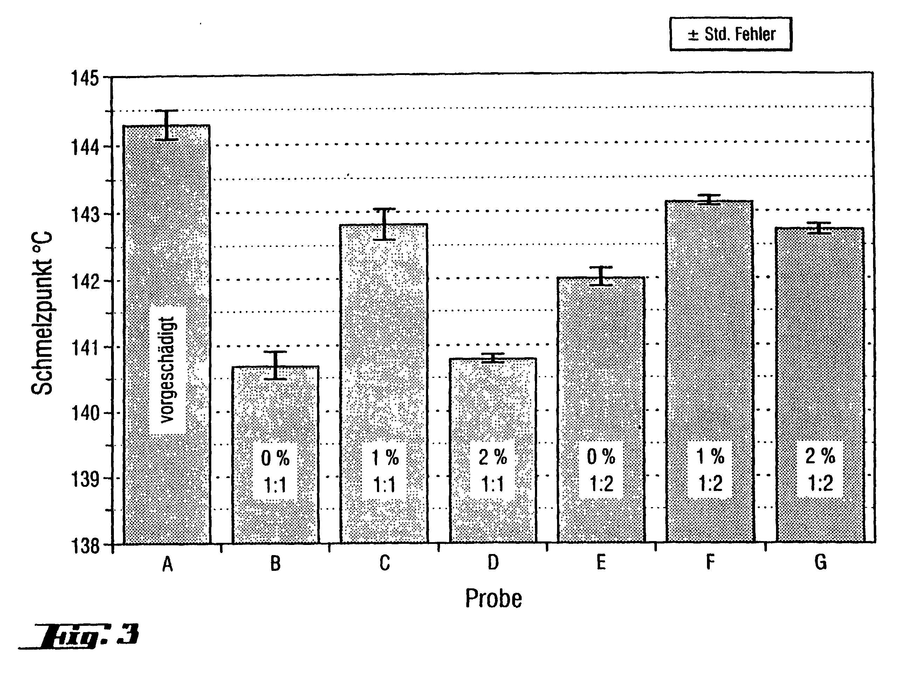

Fig. 3 gibt die Ergebnisse von vergleichenden thermoanalytischen Untersuchungen wieder, in denen strapazierte Humanhaare (Probe A), hier bezeichnet als "vorgeschadigt", neben gleichen Humanhaaren, jedoch behandelt mit einem Haarfärbemittel in Gelform ohne Pflegestoff gemäß der Erfindung (Proben B und E) bzw. behandelt mit dem entsprechenden Haarfärbemittel mit 1 oder auch 2 Gew.-% D/L-Serin als Pflegestoff (Proben C und F bzw. D und G), untersucht wurden. Die Proben B, C und D sind dabei durch Behandlung des strapazierten Haares (Probe A) mit dem fertigen Färbegel, zubereitet durch 1:1-Mischung von der entsprechenden Färbegrundlage und 1.5 %iger Wasserstoffperoxidlösung, erhalten worden, während die Proben E, F und G durch Behandlung des strapazierten Haares (Probe A) mit dem fertigen Färbegel, zubereitet durch 1:2-Mischung von der entsprechenden Färbegrundlage und 1.5 %iger Wasserstoffperoxidlösung, erhalten worden. Aus dem Diagramm (Fig. 3) ist eindeutig ersichtlich, daß in diesem Versuch durch Zusatz von D/L-Serin - bevorzugt in einer Konzentration von 1 Gew.-% - zum Haarfärbemittel die durch den Färbevorgang herbeigeführte Schmelzpunkterniedrigung des Keratins, d.h. die Strukturdestabilisierung des Keratins, stark vermindert wird.

Folgende weitere vergleichende Testreihen sollen ebenfalls die Erfindungshöhe belegen:

Es wurden Humanhaare mit Haarfärbemittel nach Beispiel 10, jedoch anstelle der dort aufgeführten die in Tabelle III genannten Verbindungen als Pflegestoffe in jeweils 2 Gew.-% enthaltend, behandelt. Die gefärbten Humanhaare wurden anschließend einer thermoanalytischen Untersuchung unterworfen. Die durch die Pflegestoffe bzw. Pflegestoff-Kombinationen hervorgerufenen Keratinschmelzpunkt-Erhöhungen (Δ Peak in °C) sind in Tabelle III angegeben, wobei als Null-Referenz die mit dem Färbemittel ohne Pflegestoffe behandelten Humanhaare (Versuch 1) dient. Es wird ersichtlich, daß in dieser Testreihe durch die zugesetzten Pflegestoffe der Erfindung eine strukturstabilisierende Wirkung erzielt wird.

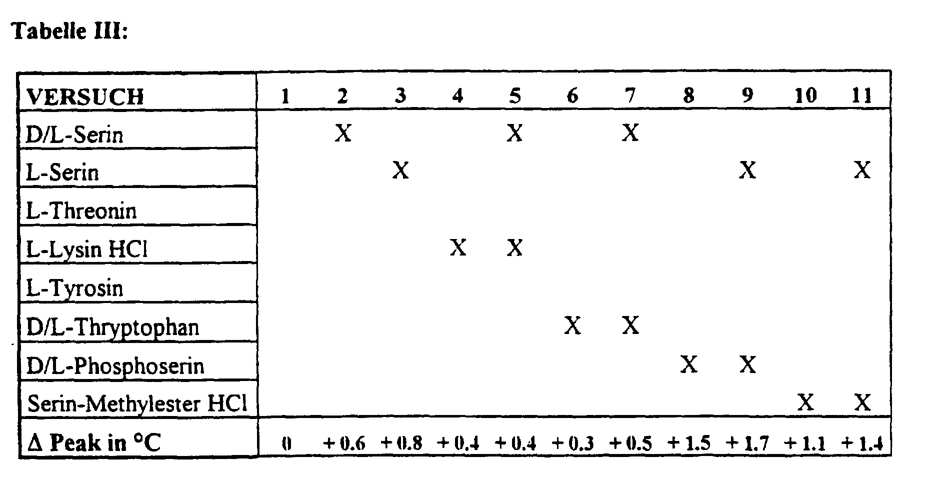

Es wurden Humanhaare mit Haarfärbemitteln nach Beispiel 11 bis 15, jedoch statt der vollständigen Pflegestoff-Kombination die Pflegestoffe einzeln und in den möglichen Zweier-Kombinationen enthaltend, behandelt und die gefärbten Humanhaare anschließend thermoanalytisch untersucht. Die resultierenden Keratinschmelzpunkt-Erhöhungen (Δ Peak in °C), bezogen auf die Haarfärbung mit Haarfärbemittel ohne Pflegestoffe gemäß Erfindung als Null-Referenz (Versuch 1), sind in Tabelle IV aufgeführt und machen deutlich, daß in dieser Versuchsreihe der Zusatz des Pflegestoffes Phospholipid EFA bzw. Panthenol allein nur zu einer geringen Strukturstabilisierung des Haares führt. Ein Vergleich der Versuche 2 und 3 mit Versuch 5 bzw. der Versuche 2,3 und 4 mit Versuch 8 in dieser Testreihe machen jedoch deutlich, daß ein starker strukturstabilisierender Effekt durch eine überraschend auftretende synergistische Wirkung der Pflegestoffe beobachtet werden kann:

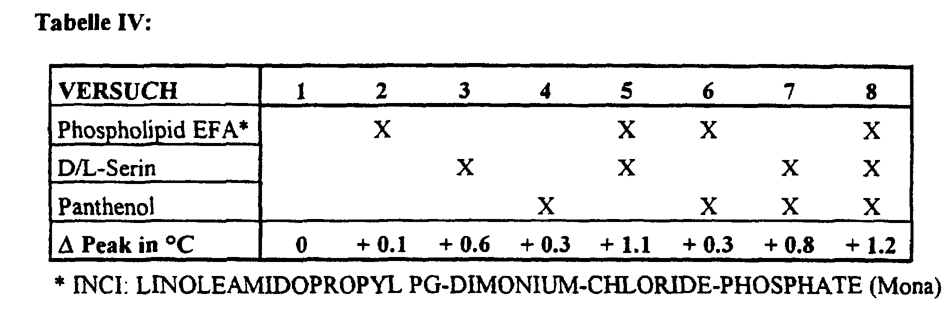

Es wurden Humanhaare mit Haarfärbemitteln nach Beispiel 16 bis 20, jedoch anstelle der vollständigen Pflegestoff-Kombination die Pflegestoffe einzeln und in den möglichen Zweier-Kombinationen enthaltend, behandelt und die gefärbten Humanhaare anschließend thermoanalytisch untersucht. Die resultierenden Keratinschmelzpunkt-Erhöhungen (Δ Peak in °C), bezogen auf die Haarfärbung mit Haarfärbemitteln ohne Pflegestoffe gemäß Erfindung (Null-Referenz), sind in Tabelle V aufgeführt:

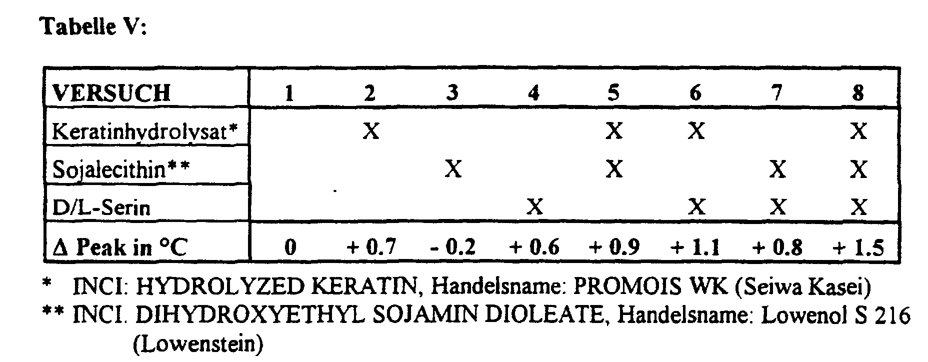

Es kann demnach in dieser Testreihe durch Zusatz der Pflegestoffe allein ein struktur-stabilisierender Effekt beobachtet werden (mit Ausnahme von Sojalecithin). Ein Vergleich der Versuche 2 und 3 mit Versuch 5 bzw. 3 und 4 mit Versuch 7 macht überraschend auftretende, synergistische Effekte durch 2er-Kombination deutlich. Versuch 8 im Vergleich zu 1 bis 7 dieser Testreihe zeigt die noch höhere, synergistische Wirkung aller drei Pflegestoffe gemeinsam.

## Patentansprüche

1. Gemisch zur oxidativen Färbung von Haaren aus
- einem Färbemittel, welches eine Kombination von mindestens einer in der Haarfärbung bekannten Entwicklersubstanz mit mindestens einer Kupplersubstanz sowie als Pflegestoff 0,01 bis 10 Gew.-% Verbindungen der allgemeinen Formel (I) enthält, 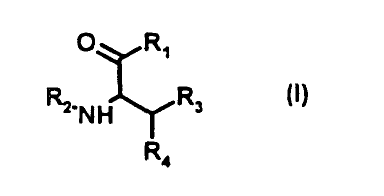 worin R¹ eine Hydroxygruppe, eine (C₁-C₃) Alkoxygruppe oder eine Gruppe der allgemeinen Formel (II) 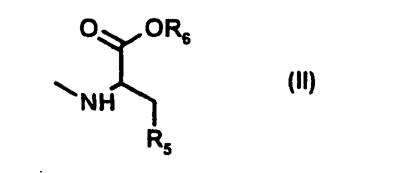 darstellt, worin R⁵ eine Hydroxygruppe, eine (CH₂)₃-NH₂-Gruppe oder eine 4-Hydroxyphenyl-Gruppe und R⁶ Wasserstoff oder eine (C₁-C₃)-Alkylgruppe sein kann, R² Wasserstoff oder eine (C₁-C₃) Alkylgruppe, R³ Wasserstoff oder eine Methylgruppe und R⁴ eine Hydroxygruppe, eine (CH₂)₃-NH₂-Gruppe, eine 4-Hydroxyphenyl-Gruppe oder eine H₂PO₃-Gruppe darstellen und deren physiologisch verträglichen Salzen von anorganischen und/oder organischen Basen, soweit die Verbindungen der allgemeinen Formel (I) bzw. (II) in der Säureform vorliegen und die allgemeine Formel (I) bzw. (II) die D- und L-Isomeren und D/L-Gemische (Racemate) umfassen, und
- einem Oxidationsmittel, ausgewählt aus Wasserstoffperoxid und dessen Anlagerungsverbindungen an Harnstoff, Melamin oder Natriumborat sowie Gemischen aus derartigen Wasserstoffperoxidanlagerungsverbindungen mit Kaliumperoxodisulfat.

2. Gemisch nach Anspruch 1, **dadurch gekennzeichnet, dass** das Färbemittel als Pflegestoff 0.01 bis 10 Gew.-% einer Verbindung der allgemeinen Formel (I), worin R¹ = OH, R² = H, R³ = H und R⁴ = OH (= Serin) bedeuten, enthält.

3. Gemisch nach Anspruch 1, **dadurch gekennzeichnet, dass** das Färbemittel als Pflegestoff eine Verbindung der allgemeinen Formel (I), worin R¹ = OH, R² = H, R³ = CH₃ und R⁴ = OH (= Threonin) bedeuten, enthält.

4. Gemisch nach Anspruch 1, **dadurch gekennzeichnet, dass** das Färbemittel als Pflegestoff eine Verbindung der allgemeinen Formel (I), worin R¹ = OH, R² = H, R³ = H und R⁴ = 4-Hydroxyphenyl (= Tyrosin) bedeuten, enthält.

5. Gemisch nach Anspruch 1, **dadurch gekennzeichnet, dass** das Färbemittel als Pflegestoff eine Verbindung der allgemeinen Formel (I), worin R¹ = OH, R² = H, R³ = H und R⁴ = (CH₂)₃NH₂ (= Lysin) bedeuten, enthält.

6. Gemisch nach Anspruch 1, **dadurch gekennzeichnet, dass** das Färbemittel als Pflegestoff eine Verbindung der allgemeinen Formel (I), worin R¹ = OCH₃, R² = H, R³ = H und R⁴ = OH (= Serin-Methylester) bedeuten, enthält.

7. Gemisch nach Anspruch 1, **dadurch gekennzeichnet, dass** das Färbemittel als Pflegestoff eine Verbindung der allgemeinen Formel (I), worin R¹ = OCH₃, R² = H, R³ = CH₃ und R⁴ = OH (= Threonin-Methylester) bedeuten, enthält.

8. Gemisch nach Anspruch 1, **dadurch gekennzeichnet, dass** das Färbemittel als Pflegestoff eine Verbindung der allgemeinen Formel (I), worin R¹ = OH, R² = H, R³ = H und R⁴ = OPO(OH₂) (= Phospho-Serin) bedeuten, enthält.

9. Gemisch nach Anspruch 1, **dadurch gekennzeichnet, dass** das Färbemittel als Pflegestoff eine Verbindung der allgemeinen Formel (I), worin R¹ = OH, R² = H, R³ = CH₃ und R⁴ = OPO(OH₂) (= Phospho-Threonin) bedeuten, enthält.

10. Gemisch nach Anspruch 1, **dadurch gekennzeichnet, dass** das Färbemittel als Pflegestoff das Serin-Dimer der allgemeinen Formeln (I) und (II), worin R¹ = allgemeine Formel (II) mit R⁵ = OH und R⁶ = H, R² = H, R³ = H und R⁴ = OH bedeuten, enthält.

11. Gemisch nach Anspruch 1, **dadurch gekennzeichnet, dass** das Färbemittel als Pflegestoff eine Kombination von 5 bis 95 Gew.-% Serin und 5 bis 95 Gew.-% Threonin enthält.

12. Gemisch nach Anspruch 1, **dadurch gekennzeichnet, dass** das Färbemittel als Pflegestoff eine Kombination von 5 bis 95 Gew.-% Serin und 5 bis 95 Gew.-% Lysin enthält.

13. Gemisch nach Anspruch 1, **dadurch gekennzeichnet, dass** das Färbemittel als Pflegestoff eine Kombination von 5 bis 95 Gew.-% Serin und 5 bis 95 Gew.-% Tyrosin enthält.

14. Gemisch nach Anspruch 1, **dadurch gekennzeichnet, dass** das Färbemittel als Pflegestoff eine Kombination von 5 bis 95 Gew.-% Threonin und 5 bis 95 Gew.-% Lysin enthält.

15. Gemisch gemäß einem der Ansprüche 1 bis 14, **dadurch gekennzeichnet, dass** das Färbemittel zusätzlich zu dem Pflegestoff oder Pflegestoffgemisch gemäß der allgemeinen Formel (I) eine oder mehrere der Aminosäuren aus der Reihe Tryptophan, Glycin, Alanin, Valin, Leucin, Isoleucin, Norleucin, Phenylalanin, Arginin, Histidin, Prolin, Hydroxyprolin, Cystin, Cystein, Methionin, Asparaginsäure, Glutaminsäure und Cysteinsäure enthält und/oder ein oder mehrere Pflegestoffe, wie Panthenol, Tocopherol, Weizenproteine, Lecithine, Keratinhydrolysate, Seidenhydrolysate, Phospholipide, Ceramide und Pseudoceramide, enthält.

16. Gemisch gemäß einem der Ansprüche 1 bis 15, **dadurch gekennzeichnet, dass** das Färbemittel Verbindungen der allgemeinen Formel (I) als Pflegestoff in der L-Form enthält.

17. Gemisch gemäß einem der Ansprüche 1 bis 16, **dadurch gekennzeichnet, dass** der pH-Wert des Färbemittels im Bereich von ungefähr 5.0 bis 12.5 und vorzugsweise im Bereich von 6.0 bis 8.0 liegt.

18. Gemisch gemäß einem der Ansprüche 1 bis 17, **dadurch gekennzeichnet, dass** das Färbemittel neben den Oxidationsfarbstoffvorprodukten auch direktziehende Farbstoffe enthält.

19. Gemisch gemäß einem der Ansprüche 1 bis 18, **dadurch gekennzeichnet, dass** das Färbemittel als Entwicklersubstanz p-Phenylendiamin, p-Toluylendiamin, 2-Hydroxyethyl-1,4-diaminobenzol, p-Aminophenol, 4-Amino-m-cresol, 4,4'-Diaminodiphenylamin und 2,4,5,6-Tetraaminopyrimidin einzeln oder im Gemisch oder andere bzw. weitere bekannte Entwicklersubstanzen enthält.

20. Gemisch nach einem der Ansprüche 1 bis 19, **dadurch gekennzeichnet, dass** das Färbemittel eine oder mehrere Kupplersubstanzen, ausgewählt aus der Gruppe α-Naphthol, Resorcin, 4-Chlorresorcin, 2-Methylresorcin, o-Aminophenol, m-Aminophenol, m-Phenylendiamin, 1,5-bzw. 2,7-Diaminonaphthalin, 4-Amino-2-hydroxytoluol, 2,4-Diamino-phenoxyethanol, 2-Amino-4-(hydroxyethyl)-aminoanisol, 3-Amino-2,4-dichlorphenol, 2-Amino-3-hydroxypyridin und 2,6-Diaminopyridin, oder andere bzw. bekannte Kupplersubstanzen enthält.

21. Gemisch gemäß Anspruch 18, **dadurch gekennzeichnet, dass** das Färbemittel als direktziehende Farbstoffe 4-Hydroxypropylamino-3-nitrophenol, HC Red No. 3, 4-Amino-3-nitrophenol, 2-Amino-6-chlor-4-nitrophenol, HC Blue No. 2, HC Blue No. 12, HC Yellow No. 2, HC Yellow No. 12, HC Red NO. 3, HC Red No. 13, HC Red No. 10 und HC Yellow No. 6 einzeln oder im Gemisch oder andere bzw. weiter bekannte Direktzieher und/oder synthetisch hergestellte oder extrahierte Naturfarbstoffe, wie Indigo ([2,2'-Bi-indolin]-3,3'-dion), Maclurin (2,3',4,4',6-Pentahydroxybenzophenon), Brasilin (C.I. Natural Red 24), Hämatoxylin, Alizarin (1,2-Dihydroxyanthrachinon), Juglon (5-Hydoxy-1,4-naphthochinon), Curcumin (2,3,4,6-Tetrahydroxy-5H-benzocyclo-6,8-heptadien-5-on), Carminsäure (C.I. Natural Red 4) oder deren chemisch abgewandelten Derivate, enthält.

22. Gemisch gemäß Ansprüche 1 bis 18, **dadurch gekennzeichnet, dass** das Färbemittel 0.5 bis 3.5 Gew.-% Pflegestoff(e) der allgemeinen Formel (I) und die in Tabelle II dargestellten bevorzugten Kombinationen von Oxidationsfarbstoffvorprodukten und gegebenenfalls direktziehenden Farbstoffen in Mengen von 0.1 bis 25 Gew.-%, bezogen auf die Gesamtmenge des kosmetischen Trägers, enthält, wobei die in Tabelle II aufgeführten Zahlen die Gew.-%-Bereiche der Oxidationsfarbstoffvorprodukte bzw. Direktzieher wiedergeben, die mit folgenden Abkürzungen erscheinen:
| | | | |
|---|---|---|---|
| A-1 | p-Toluylendiamin | B-1 | Resorcin |
| A-2 | 4-Amino-m-cresol | B-2 | α-Naphthol |
| | | B-3 | 2-Methylresorcin |
| C-1 | 4-Hydroxypropylamino-3-nitrophenol | B-4 | m-Aminophenol |
| C-2 | HC Red No. 3 | B-5 | 4-Chlorresorcin |
| C-3 | 4-Amino-3-nitrophenol | B-6 | 4-Amino-2-hydroxytoluol |
| C-4 | 2-Amino-6-chlor-4-nitrophenol | B-7 | 2-Amino-4-hydroxyethylamino-anisol |
| | | B-8 | 2-Amino-3-hydroxypyridin |

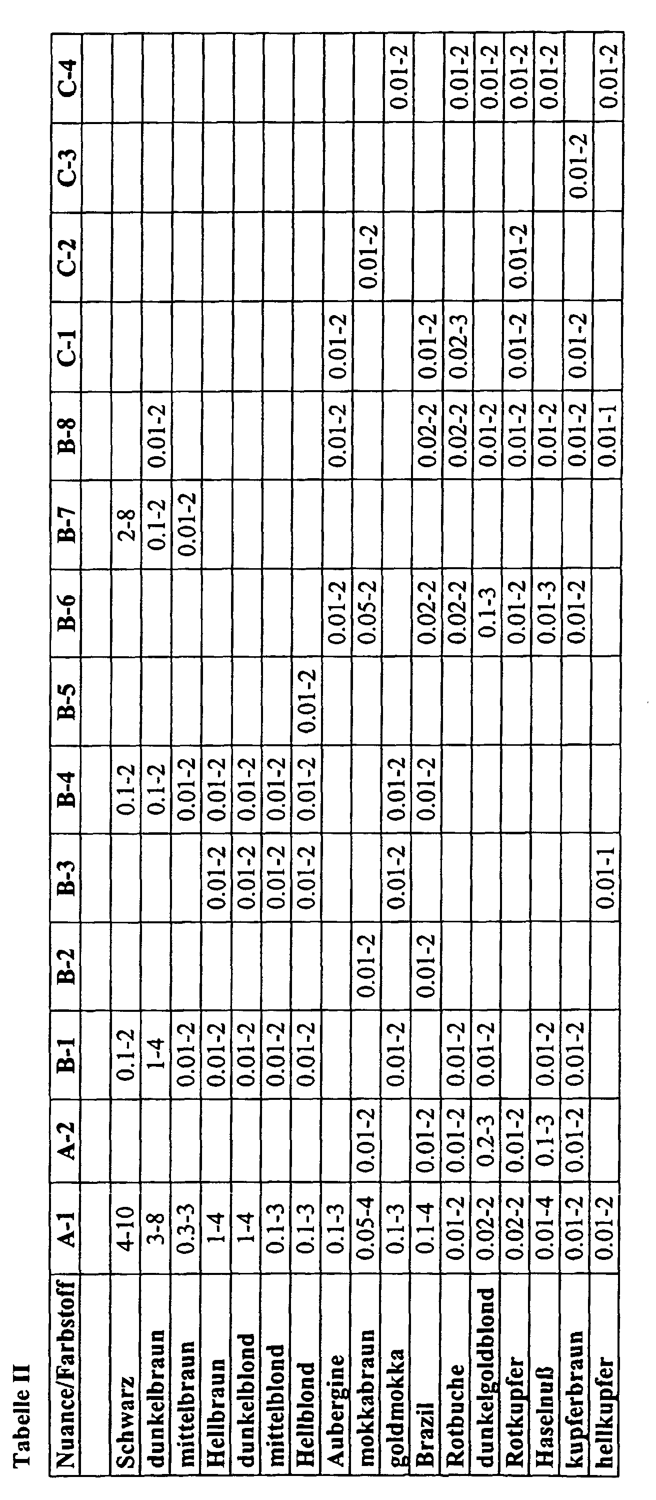

23. Gemisch nach einem der Ansprüche 1 bis 22, **dadurch gekennzeichnet, dass** das Färbemittel als Pflegestoff-Kombination 0.01 bis 10 Gew.-% Serin, 0.1 bis 5 Gew.-% Panthenol und 0.1 bis 5 Gew.-% Phospholipid EFA enthält.

24. Gemisch nach einem der Ansprüche 1 bis 22, **dadurch gekennzeichnet, dass** das Färbemittel als Pflegestoff-Kombination 0.01 bis 10 Gew.-% Serin, 0.1 bis 5 Gew.-% Keratinhydrolysat und 0.1 bis 8 Gew.-% Sojalecithin enthält.

25. Gemisch nach einem der Ansprüche 1 bis 24, **dadurch gekennzeichnet, dass** das Färbemittel als kosmetischen Träger eine wässrige Zubereitung in Form von Creme, Emulsion, Gel, Schaumaerosol, oder auch eine pulverförmige Zubereitung enthält.

26. Verwendung von 0.01 bis 10 Gew.-%, bezogen auf die Gesamtzusammensetzung der Oxidationsmittellösung (Entwicklerlotion), von Pflegestoff(en) einzeln oder im Gemisch oder Pflegestoffkombinationen nach einem oder mehreren der Ansprüche 1 bis 16 und 23 bis 24 in der Oxidationsmittellösung zur Entwicklung der Haarfärbung.

27. Verwendung von 0.01 bis 10 Gew.-%, bezogen auf die Gesamtzusammensetzung des Haarfärbemittels, von Pflegestoff(en) einzeln oder im Gemisch oder Pflegestoffkombinationen nach einem oder mehreren der Ansprüche 1 bis 16 und 23 bis 24 in fester, flüssiger oder pastöser Form als Pflegestoffsubstanz, der, direkt vor der Applikation zugesetzt, zur Vervollständigung des Haarfärbemittels dient.

28. Verfahren zum Färben von Humanhaaren, **dadurch gekennzeichnet, dass** man
A) ein Färbemittel nach einem der Ansprüche 1 bis 25, welches Pflegestoff(e) enthält, mit einer Oxidationsmittellösung (Entwicklerlotion), die gegebenenfalls Pflegestoff(e) aufweist, zum gebrauchsfertigen Haarfärbemittelbrei vermischt,
oder
B) ein Färbemittel, welches kein(e) Pflegestoff(e) enthält, mit Pflegestoff(en) vermischt, um ein Färbemittel nach einem der Ansprüche 1 bis 25 zu ergeben, und mit einer Oxidationsmittellösung (Entwicklerlotion), die gegebenenfalls Pflegestoff(e) aufweist, zum gebrauchsfertigen Haarfärbemittelbrei vermischt,
oder
C) ein Färbemittel, welches (kein(e)) Pflegestoff(e) enthält, mit einer Oxidationsmittellösung (Entwicklerlotion) in Anwesenheit von Pflegestoff(en) zum gebrauchsfertigen Gemisch nach einem der Ansprüche 1 bis 25 in Form eines Haarfärbemittelbreis verarbeitet,
D) einen nach A), B) oder C) erhaltenen Haarfärbemittelbrei auf die zu färbenden Humanhaare aufträgt und ausreichend lange bei 15 bis 40°C einwirken lässt,
E) den Haarfärbemittelbrei vom gefärbten Haar durch Spülen mit Wasser entfernt
und
F) gegebenenfalls das Haar mit einem Shampoo nachwäscht.

## Claims

1. A mixture for the oxidative colouring of hair of
- a colourant containing a combination of at least one primary intermediate known for colouring hair with at least one secondary intermediate and, as hair care component, 0.01 to 10% by weight of compounds corresponding to general formula (I): 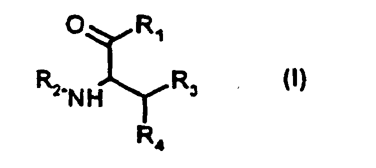 in which R¹ is a hydroxy group, a (C₁₋₃) alkoxy group or a group corresponding to general formula (II): 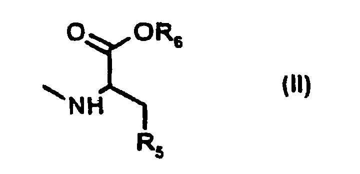 where R⁵ is a hydroxy group, a (CH₂)₃-NH₂ group or a 4-hydroxyphenyl group and R⁶ is hydrogen or a (C₁₋₃) alkyl group, R² is hydrogen or a (C₁₋₃) alkyl group, R³ is hydrogen or a methyl group and R⁴ is a hydroxy group, a (CH₂)₃-NH₂ group or a 4-hydroxyphenyl or an H₂PO₃ group,
and their physiologically compatible salts of inorganic and/or organic bases providing the compounds corresponding to general formulae (I) and (II) are present in the acid form and general formulae (I) and (II) include the D and L isomers and D/L mixtures (racemates), and
- an oxidizing component selected from hydrogen peroxide and addition compounds thereof with urea, melamine or sodium borate and mixtures of such hydrogen peroxide addition compounds with potassium peroxodisulfate.

2. A mixture as claimed in claim 1, **characterized in that** the colourant contains a compound corresponding to general formula (I), in which R¹ = OH, R² = H, R³ = H and R⁴ = OH (= serine) in a quantity of 0.01 to 10% by weight as the hair-care component.

3. A mixture as claimed in claim 1, **characterized in that** the colourant contains a compound corresponding to general formula (I) in which R¹ = OH, R² = H, R³ = CH₃ and R⁴ = OH (= threonine) as the hair-care component.

4. A mixture as claimed in claim 1, **characterized in that** the colourant contains a compound corresponding to general formula (I) in which R¹ = OH, R² = H, R³ = H and R⁴ = 4-hydroxyphenyl (= tyrosine) as the hair-care component.

5. A mixture as claimed in claim 1, **characterized in that** the colourant contains a compound corresponding to general formula (I) in which R¹ = OH, R² = H, R³ = H and R⁴ = (CH₂)₃-NH₂ (= lysine) as the hair-care component.

6. A mixture as claimed in claim 1, **characterized in that** the colourant contains a compound corresponding to general formula (I) in which R¹ = OCH₃, R² = H, R³ = H and R⁴ = OH (= serine methyl ester) as the hair-care component.

7. A mixture as claimed in claim 1, **characterized in that** the colourant contains a compound corresponding to general formula (I) in which R¹ = OCH₃, R² = H, R³ = CH₃ and R⁴ = OH (= threonine methyl ester) as the hair-care component.

8. A mixture as claimed in claim 1, **characterized in that** the colourant contains a compound corresponding to general formula (I) in which R¹ = OH, R² = H, R³ = H and R⁴ = OPO(OH₂) (= phosphoserine) as the hair-care component.

9. A mixture as claimed in claim 1, **characterized in that** the colourant contains a compound corresponding to general formula (I) in which R¹ = OH, R² = H, R³ = CH₃ and R⁴ = OPO(OH₂) (= phosphothreonine) as the hair-care component.

10. A mixture as claimed in claim 1, **characterized in that** the colourant contains the serine dimer of general formulae (I) and (II) in which R¹ = general formula (II) with R⁵ = OH and R⁶ = H, R² = H, R³ = H and R⁴ = OH as the hair-care component.

11. A mixture as claimed in claim 1, **characterized in that** the colourant contains a combination of 5 to 95% by weight of serine and 5 to 95% by weight of threonine as the hair-care component.

12. A mixture as claimed in claim 1, **characterized in that** the colourant contains a combination of 5 to 95% by weight of serine and 5 to 95% by weight of lysine as the hair-care component.

13. A mixture as claimed in claim 1, **characterized in that** the colourant contains a combination of 5 to 95% by weight of serine and 5 to 95% by weight of tyrosine as the hair-care component.

14. A mixture as claimed in claim 1, **characterized in that** the colourant contains a combination of 5 to 95% by weight of threonine and 5 to 95% by weight of lysine as the hair-care component.

15. A mixture as claimed in any of claims 1 to 14, **characterized in that**, in addition to the hair-care component or mixture of hair-care components of general formula (I), the colourant contains one or more amino acids from the group consisting of tryptophane, glycine, alanine, valine, leucine, isoleucine, norleucine, phenyl alanine, arginine, histidine, proline, hydroxyproline, cystine, cystein, methionine, aspartic acid, glutamic acid and cysteic acid and/or one or more hair-care components such as panthenol, tocopherol, wheat proteins, lecithins, keratin hydrolyzates, silk hydrolyzates, phospholipids, ceramides and pseudoceramides.

16. A mixture as claimed in any of claims 1 to 15, **characterized in that** the colourant contains compounds of general formula (I) in the L form as the hair-care component.

17. A mixture as claimed in any of claims 1 to 16, **characterized in that** the colourant has a pH value in the range from about 5.0 to 12.5 and preferably in the range from 6.0 to 8.0.

18. A mixture as claimed in any of claims 1 to 17, **characterized in that**, in addition to the oxidation dye precursors, they also contain substantive dyes.

19. A mixture as claimed in any of claims 1 to 18, **characterized in that** the colourant contains p-phenylenediamine, p-toluylenediamine, 2-hydroxyethyl-1,4-diaminobenzene, p-aminophenol, 4-amino-m-cresol, 4,4'-diaminodiphenylamine and 2,4,5,6-tetraaminopyrimidine either individually or in admixture or other known primary intermediates as the primary intermediate.

20. A mixture as claimed in any of claims 1 to 19, **characterized in that** the colourant contains one or more secondary intermediates selected from the group consisting of α-naphthol, resorcinol, 4-chlororesorcinol, 2-methyl resorcinol, o-aminophenol, m-aminophenol, m-phenylenediamine, 1,5- and 2,7-diaminonaphthalene, 4-amino-2-hydroxytoluene, 2,4-diaminophenoxyethanol, 2-amino-4-(hydroxyethyl)-aminoanisole, 3-amino-2,4-dichlorophenol, 2-amino-3-hydroxypyridine and 2,6-diaminopyridine or other known secondary intermediates.

21. A mixture as claimed in claim 18, **characterized in that** the colourant contains as substantive dyes 4-hydroxypropylamino-3-nitrophenol, HC Red No. 3, 4-amino-3-nitrophenol, 2-amino-6-chloro-4-nitrophenol, HC Blue No. 2, HC Blue No. 12, HC Yellow No. 2, HC Yellow No. 12, HC Red No. 3, HC Red No. 13, HC Red No. 10 and HC Yellow No. 6 individually or in the form of mixtures or other known substantive dyes and/or synthetic dyes or extracted natural dyes such as, for example, indigo ([2,2'-biindoline]-3,3'-dione), maclurin (2,3',4,4',6-pentahydroxybenzophenone), brasilin (C.I. Natural Red 24), hematoxylin, alizarin (1,2-dihydroxyanthraquinone), juglone (5-hydroxy-1,4-naphthoquinone), curcumin (2,3,4,6-tetrahydroxy-5H-benzocyclo-6,8-heptadien-5-one), carminic acid (C.I. Natural Red 4) or chemically modified derivatives thereof.

22. A mixture as claimed in any of claims 1 to 18, **characterized in that** the colourant contains 0.5 to 3.5% by weight of hair-care component(s) corresponding to general formula (I) and the preferred combinations shown in Table II of oxidation dye precursors and optionally substantive dyes in quantities of 0.1 to 25% by weight, based on the total quantity of the cosmetic carrier, the figures in Table II representing the % by weight ranges of the oxidation dye precursors or substantive dyes which are identified by the following codes:
| | | | |
|---|---|---|---|
| A-1 | p-toluylenediamine | B-1 | resorcinol |
| A-2 | 4-amino-m-cresol | B-2 | α-naphthol |
| | | B-3 | 2-methyl resorcinol |
| C-1 | 4-hydroxypropylamino-2-nitrophenol | B-4 | m-aminophenol |
| C-2 | HC Red No. 3 | B-5 | 4-chlororesorcinol |
| C-3 | 4-amino-3-nitrophenol | B-6 | 4-amino-2-hydroxytoluene |
| C-4 | 2-amino-6-chloro-4-nitrophenol | B-7 | 2-amino-4-hydroxyethylamino anisole |
| | | B-8 | 2-amino-3-hydroxypyridine |

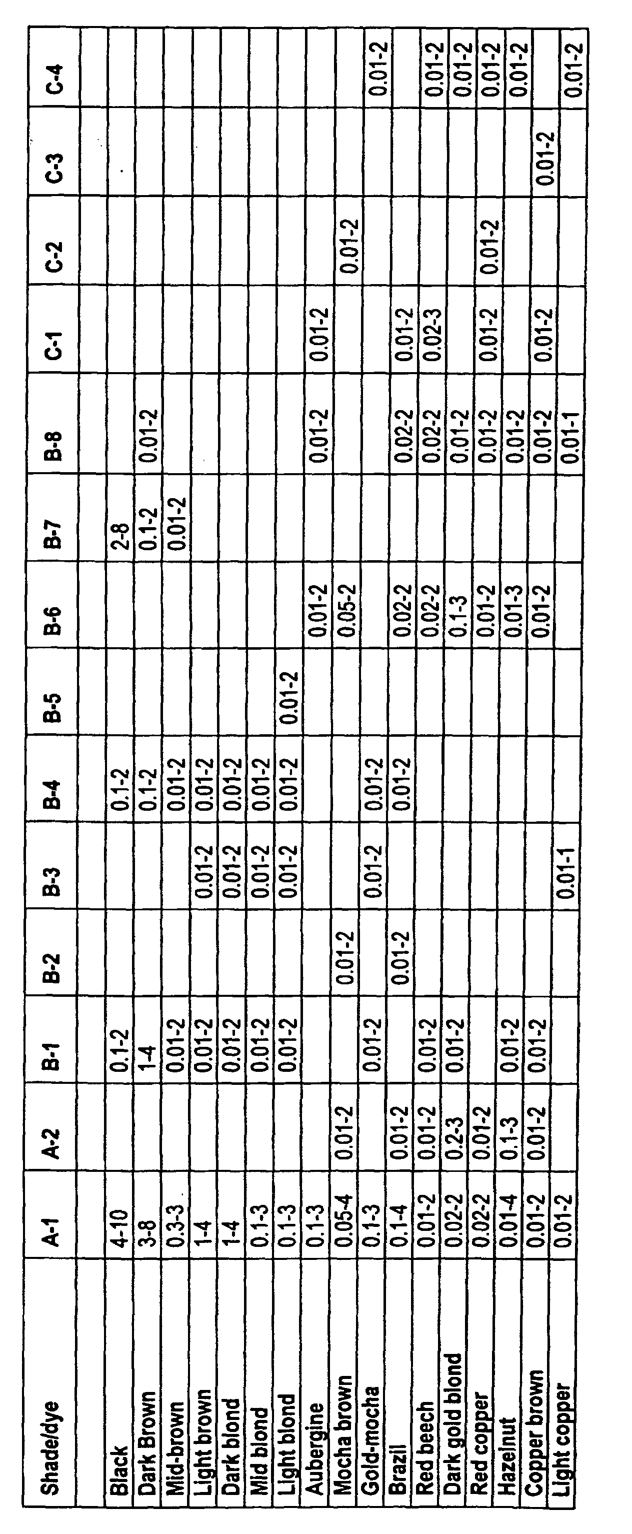

23. A mixture as claimed in any of claims 1 to 22, **characterized in that** the colourant contains a combination of 0.01 to 10% by weight of serine, 0.1 to 5% by weight of panthenol and 0.1 to 5% by weight of phospholipid EFA as the hair-care component.

24. A mixture as claimed in any of claims 1 to 22, **characterized in that** the colourant contains a combination of 0.01 to 10% by weight of serine, 0.1 to 5% by weight of keratin hydrolyzate and 0.1 to 8% by weight of soya lecithin as the hair-care component.

25. A mixture as claimed in any of claims 1 to 24, **characterized in that** the colourant contains an aqueous preparation in the form of a cream, emulsion, gel, foam aerosol or even a powder-form preparation as the cosmetic carrier.

26. The use of 0.01 to 10% by weight, based on the total composition of the oxidizing component solution (developer lotion), of hair-care component(s) either individually or in the form of a mixture or in the form of combinations according to one or more of claims 1 to 16 and 23 to 24 in the oxidizing component solution for developing the hair colour.

27. The use of 0.01 to 10% by weight, based on the total composition of the hair colourant, of hair-care component(s) individually or in the form of mixtures of combinations according to one or more of claims 1 to 16 and 23 to 24 in solid, liquid or paste-like form as a hair-care additive which, added immediately before application, serves to complete the hair colourant.

28. A process for colouring human hair, **characterized in that**
A) a colourant according to any of claims 1 to 25 which contains hair-care component(s) is mixed with an oxidizing component solution (developer lotion) optionally containing hair-care component(s) to form a ready-to-use hair colouring paste
or
B) a colourant which does not contain any hair-care component(s) is mixed with hair-care component(s) to form a hair colourant according to any of claims 1 to 25 and mixed with an oxidizing component solution (devloper lotion) optionally containing hair-care component(s) to form a ready-to-use hair colouring paste
or
C) a colourant which does (not) contain any hair-care component(s) is processed with an oxidizing component solution (developer lotion) in the presence of hair-care component(s) to form the ready-to-use mixture according to any of claims 1 to 25 in the form of a hair colouring paste,
D) the hair colouring paste obtained in accordance with A), B) or C) is applied to the human hair to be coloured and left thereon for a sufficiently long contact time at 15 to 40°C,
E) the hair colouring paste is removed from the coloured hair by rinsing with water
and
F) the hair is optionally washed with a shampoo.

## Revendications

1. Mélange pour la coloration par oxydation de cheveux, constitué
- d'un colorant, lequel contient une combinaison d'au moins une substance révélatrice connue dans la coloration capillaire avec au moins une substance de couplage ainsi qu'en tant qu'agent cosmétique, 0,01 à 10% en poids de composés de formule générale (I), 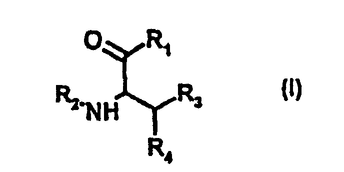 dans laquelle R¹ représente un groupe hydroxy, une groupe alcoxy en C₁ à C₃, ou un groupe de formule générale (II) 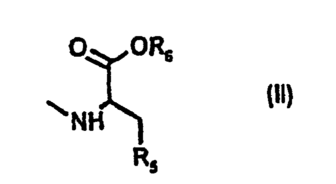 dans laquelle R⁵ peut être un groupe hydroxy, un groupe (CH₂)₃-NH₂ ou un groupe 4-hydroxyphényle et R⁶ peut être un atome d'hydrogène ou un groupe alkyle en C₁ à C₃, R² représente un atome d'hydrogène ou un groupe alkyle en C₁ à C₃, R³ représente un atome d'hydrogène ou un groupe méthyle, et R⁴ représente un groupe hydroxy, un groupe (CH₂)₃-NH₂, un groupe 4-hydroxyphényle, ou un groupe H₂PO₃ et leurs sels de bases inorganiques et/ou organiques, acceptables sur le plan physiologique, dans la mesure où les composés de formule générale (I) respectivement (II) existent sous la forme acide, et les formules générales (I) respectivement (II) comprennent les isomères D et L et les mélanges D/L (racémates) et
- un agent d'oxydation choisi parmi le peroxyde d'hydrogène et ses composés d'addition à l'urée, la mélamine ou le borate de sodium, ainsi que les mélanges de tels composés d'addition du peroxyde d'hydrogène avec le peroxodisulfate de potassium.

2. Mélange selon la revendication 1, **caractérisé en ce que** le colorant contient en tant qu'agent cosmétique, 0,01 à 10% en poids d'un composé de formule générale (I), dans laquelle R¹ = OH, R² = H, R³ = H et R⁴ = OH (= sérine).

3. Mélange selon la revendication 1, **caractérisé en ce que** le colorant contient en tant qu'agent cosmétique, un composé de formule générale (I), dans laquelle R¹ = OH, R² = H, R³ = CH₃ et R⁴ = OH (= thréonine).

4. Mélange selon la revendication 1, **caractérisé en ce que** le colorant contient en tant qu'agent cosmétique, un composé de formule générale (I), dans laquelle R¹ = OH, R² = H, R³ = H et R⁴ = 4-hydroxyphényle (= tyrosine).

5. Mélange selon la revendication 1, **caractérisé en ce que** le colorant contient en tant qu'agent cosmétique, un composé de formule générale (I), dans laquelle R¹ = OH, R² = H, R³ = H et R⁴ = (CH₂)₃-NH₂ (= lysine).

6. Mélange selon la revendication 1, **caractérisé en ce que** le colorant contient en tant qu'agent cosmétique, un composé de formule générale (I), dans laquelle R¹ = OCH₃, R² = H, R³ = H et R⁴ = OH (= ester méthylique de sérine).

7. Mélange selon la revendication 1, **caractérisé en ce que** le colorant contient en tant qu'agent cosmétique, un composé de formule générale (I), dans laquelle R¹ = OCH₃, R² = H, R³ = CH3 et R⁴ = OH (= ester méthylique de thréonine).

8. Mélange selon la revendication 1, **caractérisé en ce que** le colorant contient en tant qu'agent cosmétique, un composé de formule générale (I), dans laquelle R¹ = OH, R² = H, R³ = H et R⁴ = OPO(OH₂) (= phosphosérine).

9. Mélange selon la revendication 1, **caractérisé en ce que** le colorant contient en tant qu'agent cosmétique, un composé de formule générale (I), dans laquelle R¹ = OH, R² = H, R³ = CH₃ et R⁴ = OPO(OH₂) (= phosphothréonine).

10. Mélange selon la revendication 1, **caractérisé en ce que** le colorant contient en tant qu'agent cosmétique, le dimère de sérine des formules générales (I) et (II), dans lesquelles R¹ = la formule générale (II) avec R⁵ = OH et R⁶ = H, R² = H, R³ = H et R⁴ = OH.

11. Mélange selon la revendication 1, **caractérisé en ce que** le colorant contient en tant qu'agent cosmétique, une combinaison de 5 à 95% en poids de sérine et de 5 à 95% en poids de thréonine.

12. Mélange selon la revendication 1, **caractérisé en ce que** le colorant contient en tant qu'agent cosmétique, une combinaison de 5 à 95% en poids de sérine et de 5 à 95% en poids de lysine.

13. Mélange selon la revendication 1, **caractérisé en ce que** le colorant contient en tant qu'agent cosmétique, une combinaison de 5 à 95% en poids de sérine et de 5 à 95% en poids de tyrosine.

14. Mélange selon la revendication 1, **caractérisé en ce que** le colorant contient en tant qu'agent cosmétique, une combinaison de 5 à 95% en poids de thréonine et de 5 à 95% en poids de lysine.

15. Mélange selon l'une quelconque des revendications 1 à 14, **caractérisé en ce que** le colorant contient en complément à l'agent cosmétique ou au mélange d'agents cosmétiques selon la formule générale (I), un ou plusieurs des acides aminés de la série, tryptophane, glycine, alanine, valine, leucine, isoleucine, norleucine, phénylalanine, arginine, histidine, proline, hydroxyproline, cystine, cystéine, méthionine, acide aspartique, acide glutamique, et acide cystéique, y sont contenus et/ou un ou plusieurs agents cosmétiques, tels que le panthénol, le tocophérol, les protéines du blé, les lécithines, les hydrolysats de kératine, les hydrolysats de soie, les phospholipides, les céramides et les pseudocéramides.

16. Mélange selon l'une quelconque des revendications 1 à 15, **caractérisé en ce que** le colorant contient des composés de formule générale (I) sous la forme L en tant qu'agent cosmétique.

17. Mélange selon l'une quelconque des revendications 1 à 16, **caractérisé en ce que** la valeur du pH du colorant est dans la plage d'environ 5,0 à 12,5 et de préférence dans la plage de 6,0 à 8,0.

18. Mélange selon l'une quelconque des revendications 1 à 17, **caractérisé en ce que** le colorant contient, en plus des précurseurs de colorant par oxydation, également des colorants montant directement sur la fibre.

19. Mélange selon l'une quelconque des revendications 1 à 18, **caractérisé en ce que** le colorant contient en tant que substance révélatrice, la p-phénylènediamine, la p-toluylènediamine, le 2-hydroxyéthyl-1,4-diaminobenzène, le p-aminophénol, le 4-amino-m-crésol, la 4,4'-diaminodiphénylamine, et la 2,4,5,6-tétraaminopyrimidine, individuellement ou en mélange, ou des substances révélatrices autres respectivement connues supplémentaires.

20. Mélange selon l'une quelconque des revendications 1 à 19, **caractérisé en ce que** le colorant contient une ou plusieurs substances de couplage, choisies dans le groupe formé par l'α-naphtol, la résorcine, la 4-chlororésorcine, la 2-méthylrésorcine, l'o-aminophénol, le m-aminophénol, la m-phénylènediamine, le 1,5- ou 2,7-diaminonaphtalène, le 4-amino-2-hydroxytoluène, le 2,4-diamino-phénoxyéthanol, le 2-amino-4-(hydroxyéthyl)-aminoanisole, le 3-amino-2,4-dichlorophénol, la 2-amino-3-hydroxypyridine, et la 2,6-diaminopyridine, ou des substances de couplage autres respectivement connues.

21. Mélange selon la revendication 18, **caractérisé en ce que** le colorant contient en tant que colorants montant directement sur la fibre, le 4-hydroxypropylamino-3-nitrophénol, HC Red N°3, le 4-amino-3-nitrophénol, le 2-amino-6-chloro-4-nitrophénol, HC Blue N°2, HC Blue N°12, HC Yellow N°2, HC Yellow N°12, HC Red N°3, HC Red N°13, HC Red N°10 et HC Yellow N°6, individuellement ou en mélange ou des substances montant directement sur la fibre, autres respectivement connues supplémentaires et/ou des colorants naturels extraits ou préparés par synthèse, tels que l'indigo (la [2,2'-bi-indoline]-3,3'-dione), la maclurine (la 2,3',4,4',6-pentahydroxybenzo-phénone), la brasiline (C.I. Natural Red 24), l'hématoxyline, l'alizarine (la 1,2-dihydroxyanthraquinone), la juglone (la 5-hydroxy-1,4-naphtoquinone), la curcumine (la 2,3,4,6-tétrahydroxy-5H-benzocyclo-6,8-heptadiène-5-one), l'acide carminique (C.I. Natural Red 4) ou leurs dérivés chimiquement transformés.

22. Mélange selon les revendications 1 à 18, **caractérisé en ce que** le colorant contient 0,5 à 3,5% en poids d'agent(s) cosmétique(s) de formule générale (I) et les combinaisons préférées, représentées dans le tableau II, des précurseurs de colorant par oxydation, et éventuellement les colorants montant directement sur la fibre en des quantités de 0,1 à 25% en poids, par rapport à la quantité totale du véhicule cosmétique, les chiffres énumérés dans le tableau Il indiquent les plages des pourcents en poids des précurseurs de colorants par oxydation, respectivement des substances montant directement sur la fibre, qui apparaissent avec les abréviations suivantes :
| | | | |
|---|---|---|---|
| A-1 | p-toluylènediamine | B-1 | résorcine |
| A-2 | 4-amino-m-crésol | B-2 | α-naphtol |
| | | B-3 | 2-méthylrésorcine |
| C-1 | 4-hydroxypropylamine-3-nitrophénol | B-4 | m-aminophénol |
| C-2 | HC Red N° 3 | B-5 | 4-chlororésorcine |
| C-3 | 4-amino-3-nitrophénol | B-6 | 4-amino-2-hydroxytoluène |
| C-4 | 2-amino-6-chloro-4-nitrophénol | B-7 | 2-amino-4-hydroxyéthylamino-anisol |
| | | B-8 | 2-amino-3-hydroxypyridine |

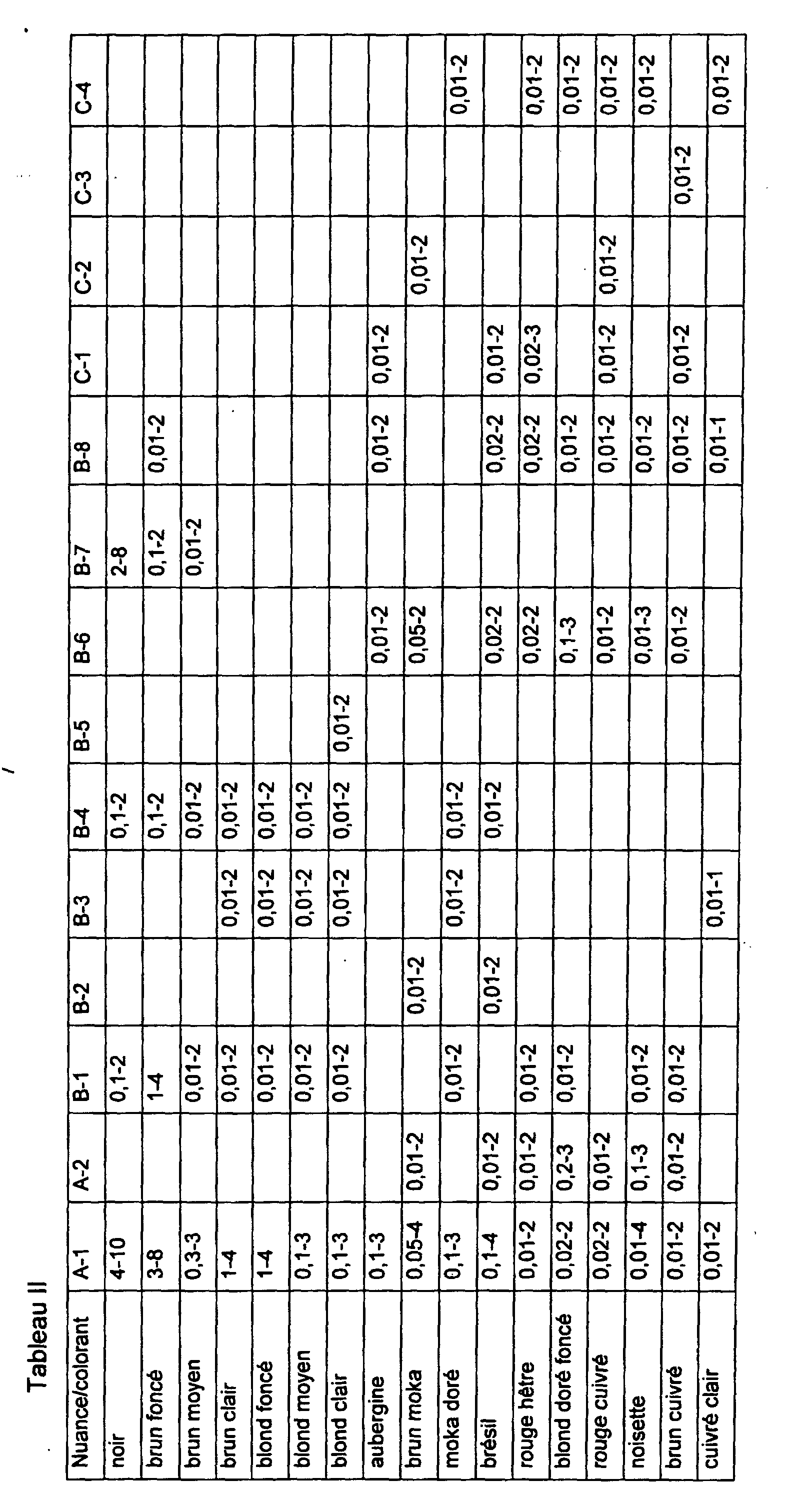

23. Mélange selon l'une quelconque des revendications 1 à 22, **caractérisé en ce que** le colorant contient en tant que combinaison d'agents cosmétiques, 0,01 à 10% en poids de sérine, 0,1 à 5% en poids de panthénol, et 0,1 à 5% en poids de phospholipide EFA.

24. Mélange selon l'une quelconque des revendications 1 à 22, **caractérisé en ce que** le colorant contient en tant que combinaison d'agents cosmétiques, 0,01 à 10% en poids de sérine, 0,1 à 5% en poids d'hydrolysat de kératine, et 0,1 à 8% en poids de lécithine de soja.

25. Mélange selon l'une quelconque des revendications 1 à 24, **caractérisé en ce que** le colorant contient une préparation aqueuse sous forme de crème, émulsion, gel, aérosol moussant, ou également une préparation pulvérulente, en tant que véhicule cosmétique.

26. Utilisation de 0,01 à 10% en poids, par rapport à la composition totale de la solution d'agent d'oxydation (lotion révélatrice), d'agent(s) cosmétique(s), individuels ou en mélange ou bien de combinaisons d'agents cosmétiques selon l'une ou plusieurs des revendications 1 à 16 et 23 à 24, dans la solution d'agent d'oxydation pour révéler la coloration capillaire.

27. Utilisation de 0,01 à 10% en poids, par rapport à la composition totale du colorant capillaire, d'agent(s) cosmétique(s), individuels ou en mélange, ou bien de combinaisons d'agents cosmétiques selon l'une ou plusieurs des revendications 1 à 16 et 23 à 24, sous une forme solide, liquide, ou pâteuse, en tant que substance cosmétique, qui, ajoutée directement avant l'application, sert à compléter le colorant capillaire.

28. Procédé de coloration de cheveux humains, **caractérisé en ce que**,
A) un colorant selon l'une quelconque des revendications 1 à 25, qui contient un ou des agents cosmétiques, est mélangé avec une solution d'agent d'oxydation (lotion révélatrice) qui présente éventuellement un ou des agents cosmétiques, afin d'obtenir un coulis de colorant capillaire prêt à l'emploi,
Ou
B) un colorant, qui ne contient pas d'agent(s) cosmétique(s), est mélangé avec un ou des agents cosmétiques, afin d'obtenir un colorant capillaire selon l'une quelconque des revendications 1 à 25, avec une solution d'agent d'oxydation (lotion révélatrice), qui présente éventuellement un ou des agents cosmétiques, afin d'obtenir un coulis de colorant capillaire prêt à l'emploi,
ou
C) un colorant, qui contient ou ne contient pas d'agent(s) cosmétique(s), qui est transformé avec une solution d'agent d'oxydation (lotion révélatrice) en présence d'un ou des agents cosmétiques, pour le mélange prêt à l'emploi selon l'une quelconque des revendications 1 à 25 sous forme d'un coulis de colorant capillaire,
D) un coulis de colorant capillaire obtenu selon A), B) ou C) est appliqué sur les cheveux humains à colorer, et est laissé suffisamment longtemps à une température de 15 à 40°C,
E) le coulis de colorant capillaire est éliminé du cheveu coloré, par rinçage à l'eau,
et
F) le cheveu est éventuellement lavé ultérieurement avec un shampoing.
